Europäisches Patentamt

European Patent Office

Office européen des brevets

(11) Publication number: **0 168 214 B1**

(19)

(12) **EUROPEAN PATENT SPECIFICATION**

(45) Date of publication of patent specification: **26.10.94**

(21) Application number: **85304758.7**

(22) Date of filing: **03.07.85**

(51) Int. Cl.⁵: **C07K 3/18**, C07K 13/00, A61K 37/02, C12P 21/02, C12N 15/00, C12Q 1/68, A61K 37/66, //(A61K37/66, 37:02)

(54) **Tumor necrosis factor, methods for its preparation, compositions containing it, DNA encoding it and assay method using such DNA.**

(30) Priority: **05.07.84 US 628059**
**05.07.84 US 627959**
**05.07.84 US 628060**
**03.12.84 US 677454**
**03.12.84 US 677156**
**03.12.84 US 677257**

(43) Date of publication of application:
**15.01.86 Bulletin 86/03**

(45) Publication of the grant of the patent:
**26.10.94 Bulletin 94/43**

(84) Designated Contracting States:
**AT BE CH DE FR GB IT LI LU NL SE**

(56) References cited:
**EP-A- 0 131 789     EP-A- 0 146 026**
**EP-A- 0 148 311     EP-A- 0 155 549**
**EP-A- 0 158 286     CH-A- 65 623**
**DE-A- 3 421 731     GB-A- 2 094 833**

(73) Proprietor: **GENENTECH, INC.**
**460 Point San Bruno Boulevard**
**South San Francisco California 94080 (US)**

(72) Inventor: **Aggarwal, Bharat Bhushan**
**324 Del Rosa Way**
**San Mateo California 94403 (US)**
Inventor: **Lee, Sang He**
**1035 San Carlos Avenue**
**El Granada California 94018 (US)**
Inventor: **Goeddel, David Vannorman**
**2115 Forestview**
**Hillsborough California 94010 (US)**
Inventor: **Nedwin, Glenn Evan**
**429 S. Hoop Pole Road**
**Guilford Connecticut 06437 (US)**

(74) Representative: **Armitage, Ian Michael et al**
**MEWBURN ELLIS**
**York House**
**23 Kingsway**
**London WC2B 6HP (GB)**

JOURNAL OF IMMUNOLOGY, vol. 125, no. 4, October 1980, pages 1671-1677; The Wiliiams & Wilkins Co., US. M.R. RUFF et al.: "Purification and physicochemical characterization of rabbit tumor necrosis factor."

CHEMICAL ABSTRACTS, vol. 102, no. 15, April 15, 1985, page 473, ref. no.130173u; Columbus, Ohio, US B.B. AGGARWAL et al.

Cell Immun. 38, 388-402

PNAS USA Vol. 82, 6637-6641

NATURE, vol. 312, no. 5996, December 20/27, 1984, pages 724-729; London, GB D. PENNICA et al.: "Human tumour necrosis factor: precursor structure, expression and homology to lymphotoxin".

PNAS USA Vol. 80, 5397-5401

Nature 312, 721-724

**Description**

This application relates to lymphokines. In particular, it relates to cytotoxic factors secreted by lymph cells and methods for making same in recombinant cells.

Immune cells such as B cells, T Cells, natural killer cells and macrophages are known to elaborate substances that exert cytotoxic activity toward tumor cells but which are innocuous to normal cells. These substances have been variously named, for example, lymphotoxin, tumor necrosis factor, NK cell cytotoxic factor, hemorrhagic necrosis factor, macrophage cytotoxin or macrophage cytotoxic factor. At the present time the identities of the proteins associated with these names are unclear. The principal difficulties have been that the biological assays employed to detect the proteins do not discriminate among them, the proteins appear to be found in nature as aggregates or hydrolytic products, and the amounts heretofore obtained have been so small that the high degree of purification needed to fully characterize the proteins has not been reached.

Typically, such cytotoxic substances are found in the sera of intact animals, or in the culture supernatants of lymph cells or cell lines after the animals or cells had been treated with a substance known to stimulate the proliferation of immune cells (an "inducer"). Thereafter the serum or supernatant is recovered and assayed for cytotoxic activity towards a target tumor cell line. A standard target is L-929, a murine tumor cell line. This cell line and others used in bioassays of this type are nonspecific in their lytic response because a variety of apparently discrete lymph cell products are able to effect lysis. Similar nonspecific responses are observed in in vitro tumor necrosis assays. Thus, cytolytic assays which observe for the lysis of cell lines in vitro or tumor necrosis in vivo are inadequate to distinguish among the various cytotoxic lymph products.

Cytotoxic factors tentatively have been classified on the basis of the lymph cells from which they are induced. For example, lymphotoxin is a name commonly applied to the cytotoxic secretory products of B or T lymphocytes, or cell lines derived therefrom, while tumor necrosis factor often is used to describe cytotoxic products of macrophages or their derived cell lines. This classification system, however, has not been developed to the point where there is any assurance that only a single protein is being referred to, or that proteins referred to by different name are in fact different.

Attempts have been made to purify and characterize the cytotoxic factors secreted by each cell type. To the extent that reports vary as to a property of a cytotoxic factor, or are completely inconsistent as to a given property, it could be concluded either that the characterization was erroneous or that a plurality of discrete cytotoxic factors are secreted by each cell type. For example, the cytotoxic products derived from macrophages, monocytes or monocytic cell lines, while sometimes generally referred to as tumor necrosis factor, have been reported to have properties that appear inconsistent with a theory of a single cytotoxic product. See for example the following literature: R. MacFarlan et al., 1980, "AJEBAK" 58(pt 5): 489-500; D. Mannel et al., 1980, "Infection and Immunology" 30(2): 523-530; H. Ohnishi et al., UK patent application 2,106,117A; and J. Hammerstrom, 1982, "Scand J. Immunol." 15: 311-318.

On the other hand C. Zacharchuk et al., 1983, "Proc. Nat. Acad. Sci. USA", 80: 6341-6345 suggest that guinea pig lymphotoxin and a cytotoxic factor from guinea pig macrophages are immunochemically similar, if not identical. Similar conclusions are advanced in Ruff et al., 1981, Lymphokines Vol. 2 pp 235-272 at pp 241-242.

The attempts at characterization of immune cytotoxic factors also have focused on using as starting material the sera or peritoneal fluid of animals that have been exposed to immunogenic antigens. These sources contain the entire cornucopia of the stressed immune system, in contrast to the product or products of a single cell type or line. The following should be consulted as examples of publications of this type: S. Green et al., 1982, "J. Nat. Cancer Inst." 68(6): 997-1003 ("tumor necrosis-inducing factor"); M. Ruff et al., 1980, "J. Immunology" 125(4): 1671-1677 ("tumor necrosis factor"); H. Enomoto et al., European Patent Application 86475 ("antitumor substance"); H. Oettgen et al., 1980, "Recent Results Cancer Res." 75: 207-212 ("tumor necrosis factor"); F. Kull et al., 1981, "J. Immunol." 126(4): 1279-1283 ("Tumor Cell Cytotoxin:); D. Mannel et al., 1980, "Infection and Immunity" 28(1): 204-211 ("cytotoxic factor"); N. Matthews et al., 1980, "Br. J. Cancer: 42: 416-422 ("tumor necrosis factor"); S. Green et al., 1976, "Proc. Nat. Acad. Sci. USA; 73(2): 381-385 ("serum factor"); N. Satomi et al., 1981, "Jpn J. Exp. Med." 51(6): 317-322; N. Matthews, 1979, "Br. J. Cancer" 40: 534-539 ("tumor necrosis factor"); K. Haranaka et al., 1981, "Jpn. J. Exp. Med." 51(3): 191-194 ("tumor necrosis factor"); and L. Old et al., European Patent Application 90892; T. Umeda et al., 1983, "Cellular and Molecular Biology" 29(5): 349-352; and H. Enomoto et al., 1983, European Patent Application 86,475.

Further literature which should be consulted is J. Nissen-Meyer et al., 1982, "Infection and Immunity" 38(1): 67-73; J. Klostergaard et al., 1981, "Mol. Immunol." 18(12): 1049-1054; N. Sloane, U.S. patent 4,359,415; and H. Hayashi et al., U.S patent 4,447,355; K. Hanamaka et al., 1983, European Patent Application 90,892; and G. Granger et al., 1978, "Cellular Immunology" 38: 388-402.

European Patent Application Publn. No. 100641 describes a cytotoxic polypeptide which was purified substantially free of impurities from a human lymphoblastoid cell culture. This polypeptide was designated lymphotoxin, although its relationship to other reported cytotoxic polypeptides under the name lymphotoxin is conjectural. It was not known whether this was the sole cytotoxic polypeptide elaborated by immune cells, as suggested by Zacharchuk et al. (Id.), or whether it was one of a potential family of cytotoxic factors.

The polypeptide of the '641 Application has two amino termini, a larger variant ending with Leu Pro Gly Val Gly Leu Thr Pro Ser Ala Ala Gln Thr Ala Arg Gln His Pro Lys Met His Leu Ala His Ser Thr.... and a smaller variant with the truncated amino terminus His Ser Thr Leu Lys Pro Ala Ala ...

According to the prior literature the interferons, which exhibit some tumor inhibitory activity, and a poorly characterized protein having an AlaAla amino terminus (U.K. Patent Application Publn. No. 2,117,385A), were candidates for non-lymphotoxin cytotoxic factors. As will be seen, the tumor necrosis factor of this invention is not an interferon, is not lymphotoxin and does not have an AlaAla amino terminus.

In Proc. Natl. Acad. Sci 80,5397-5401 (September '83) Williamson et al describe the production from human B-cell lines of a cytotoxic factor of molecular weight 70 000 which they designate "tumor necrosis factor (TNF)" and which exhibits a synergistic cytotoxic effect in combination with interferon. The monocytic and promyelocyte cell lines tested which included the cell line HL-60 produced little or none of the cytotoxic factor.

Embodiments of the invention make it possible to attain one or more of the following objects:

(a) to conclusively determine whether or not another tumor necrosis factor than lymphotoxin exists and, if so, to identify it in such a way as to clearly distinguish other such factors;

(b) to produce such a factor by methods other than induced cell culture, which is expensive and yields product which is contaminated with the inducing agent, or by induction of peripheral blood lymphocytes, which is economically impractical, poorly reproducible, and produces product contaminated with homologous cellular and plasma proteins;

(c) to obtain DNA encoding such tumor necrosis factor and to express the DNA in recombinant culture;

(d) to synthesize such factor in recombinant culture in the mature form;

(e) to modify the coding sequence or structure of such factor;

(f) to formulate such factor into therapeutic dosage forms and to administer same to animals for the treatment of tumors; and

(g) to produce diagnostic reagents relating to such factor.

Thus in a first aspect the invention provides a method for preparing a tumor necrosis factor from an admixture with other proteins, comprising adsorbing the tumor necrosis factor from such admixture onto a substance selected from:

(i) a silicate,

(ii) controlled pore glass,

(iii) alkyl sepharose

(iv) particles of an anion exchange resin having substantially uniform particle size,

and thereafter eluting the tumor necrosis factor.

In a second aspect the invention provides human tumor necrosis factor as defined in claims 4-6. In further aspects the invention provides tumor necrosis factor ("TNF") as defined in claims 7-17; DNA encoding such TNF; cells transformed with the DNA; and compositions comprising the TNF optionally including an interferon.

A cytotoxic factor has been purified to homogeneity, characterized and expressed in recombinant culture. This factor is designated tumor necrosis factor (TNF) for convenience and is defined below. It is provided in substantially homogeneous form from cell culture at a specific activity of greater than about 10 million units/mg protein, and ordinarily about 100 million units/mg.

Human tumor necrosis factor synthesized in recombinant culture is characterized by the presence of non-human cell components, including proteins, in amounts and of a character which are physiologically acceptable for administration to patients in concert with the tumor necrosis factor. These components ordinarily will be of yeast, procaryotic or non-human higher eukaryotic origin and present in innocuous contaminant quantities, on the order of less than about 1 percent by weight. Further, recombinant cell culture enables the production of tumor necrosis factor absolutely free of homologous proteins. Homologous proteins are those which are normally associated with the tumor necrosis factor as it is found in nature, e.g. in cells, cell exudates or body fluids. For example, a homologous protein for human tumor necrosis factor is human serum albumin. Heterologous proteins are the converse, i.e. they are not naturally associated or found in combination with the tumor necrosis factor in question.

As specified in the claims hereof, DNA is provided that encodes tumor necrosis factor and which, when expressed in recombinant or transformed culture, yields copious quantities of tumor necrosis factor.

EP 155549A, mentioned under Art. 54(3) EPC, discloses the deduced amino acid sequence for a precursor TNF resulting from cloning cDNA derived from human cells, and its expression in a recombinant host. Various modifications of the amino acid sequence are proposed, but none which specifies the N-terminus of mature human TNF as disclosed in the present application.

Chromosomal DNA encoding TNF is obtained by probing genomic DNA libraries with cDNA. Chromosomal DNA is free of flanking regions encoding other proteins but may contain introns.

The isolated tumor necrosis factor DNA is readily modified by substitution, deletion or insertion of nucleotides, thereby resulting in novel DNA sequences encoding tumor necrosis factor or its derivatives. These modified sequences are used to produce mutant tumor necrosis factor and to directly express mature tumor necrosis factor. The modified sequences also are useful in enhancing the efficiency of tumor necrosis factor expression in chosen host-vector systems, e.g. by modification to conform to a host cell codon preference.

These novel DNA sequences or fragments thereof are labelled and used in hybridization assays for genetic material encoding tumor necrosis factor.

In processes for the synthesis of tumor necrosis factor, DNA which encodes tumor necrosis factor is ligated into a replicable (reproducible) vector, the vector used to transform host cells, the host cells cultured and tumor necrosis factor recovered from the culture. This general process is used to construct tumor necrosis factor having the characteristics of monocyte tumor necrosis factor or to construct novel derivatives of tumor necrosis factor, depending upon vector construction and the host cell chosen for transformation. The tumor necrosis factor species which are capable of synthesis herein include mature (valyl amino-terminal) tumor necrosis factor, pretumor necrosis factor ("preTNF", defined herein), and derivatives of TNF including (a) fusion proteins wherein TNF or any fragment thereof (including mature tumor necrosis factor) is linked to other proteins or polypeptides by a peptide bond at the amino and/or carboxyl terminal amino acids of TNF or its fragments, (b) TNF fragments, including mature tumor necrosis factor or fragments of preTNF in which any preprotein amino acid is the amino-terminal amino acid of the fragment, (c) mutants of TNF or its fragments (including mature tumor necrosis factor) wherein one or more amino acid residues are substituted, inserted or deleted, and/or (d) methionyl or modified methionyl (such as formyl methionyl or other blocked methionyl species) amino-terminal addition derivatives of the foregoing proteins, fragments or mutants.

Ordinarily, if a mammalian cell is transformed with (a) a vector containing the entire tumor necrosis factor structural gene (including a 5' start codon), or (b) the gene for mature tumor necrosis factor or a tumor necrosis factor derivative operably ligated to a eukaryotic secretory leader (which may also include the tumor necrosis factor secretory leader presequence), and the cell cultured, then mature tumor necrosis factor is recovered from the culture.

Similarly, if DNA which encodes TNF is operably ligated in a vector to a secretory leader which is properly processed by the host cell to be transformed (usually the organism from which the leader sequence was obtained), the host transformed with the vector and cultured, then the tumor necrosis factor is synthesized without amino-terminal methionyl or blocked methionyl. In particular, E. coli transformed with vectors in which DNA encoding mature tumor necrosis factor is ligated 5' to DNA encoding the STII enterotoxin signal polypeptide will properly process a high percentage of the hybrid preprotein to mature tumor necrosis factor. Secretory leaders and host cells may be selected that also result in proper transport of mature protein into cell periplasm.

Also within the scope of this invention are derivatives of tumor necrosis factor other than variations in amino acid sequence or glycosylation. Such derivatives are characterized by covalent or aggregative association with chemical moieties. The derivatives generally fall into three classes: salts, side chain and terminal residue covalent modifications, and adsorption complexes.

If DNA encoding mature tumor necrosis factor is operably ligated into a vector, the vector used to transform a host cell and the cell cultured, mature tumor necrosis factor is found in the cell cytoplasm. Accordingly, it is unnecessary to devise secretion systems in order to obtain mature tumor necrosis factor. This was surprising because, ordinarily, direct expression yields methionylated protein. Further, the protein is stable and soluble in recombinant cell culture, i.e., it is neither proteolytically cleaved by intracellular proteases nor deposited as refractile bodies. Accordingly, novel fermentations are provided that comprise lower eukaryotic or prokaryotic cells having unmethionylated mature tumor necrosis factor located within the cytoplasm of such cells.

While tumor necrosis factor may be prepared by culturing animal cell lines, e.g. a monocytic cell line induced by growth in the presence of 4-beta-phorbol-12-myristate-13-acetate (PMA) or immortal cell lines such as hybridomas or EBV transformed cells (U.S. Patent 4,464,465), it is preferable to synthesize tumor necrosis factor in recombinant cell culture as described further below.

Once tumor necrosis factor is prepared by fermentation it generally is purified by recovering the supernatant culture fluid or lysed cell culture, removing solids, adsorbing tumor necrosis factor from the supernatant admixture (containing tumor necrosis factor and other proteins) onto a hydrophobic substance, eluting tumor necrosis factor from the substance, adsorbing tumor necrosis factor onto a tertiary amino anion exchange resin, eluting tumor necrosis factor from the resin, adsorbing tumor necrosis factor onto an anion exchange resin (preferably quaternary amino-substituted) having substantially uniform particle size, and eluting tumor necrosis factor from the resin. Optionally, the tumor necrosis factor compositions are concentrated and purified by chromatofocusing at any point in the purification procedure, for example by isoelectric focusing or passage through a sieving gel such as Sephadex G-25.

The purified tumor necrosis factor from recombinant or induced cell culture is combined for therapeutic use with physiologically innocuous stabilizers and excipients and prepared in dosage form as by lyophilization in dosage vials or storage in stabilized aqueous preparations. Alternatively, tumor necrosis factor is incorporated into a polymer matrix for implantation into tumors or surgical sites from which tumors have been excised, thereby effecting a timed-release of the tumor necrosis factor in a localized high gradient concentration.

The compositions herein are obtained free of contaminant cytotoxic factors such as lymphotoxin, interferons or other cytotoxic proteins referred to in the literature. However, in therapeutic applications tumor necrosis factor is advantageously combined with predetermined amounts of lymphotoxin and/or interferon. Compositions containing tumor necrosis factor and an interferon such as gamma interferon are particularly useful since they have been found to exert a synergistic cytotoxic activity.

Tumor necrosis factor compositions are administered to animals, particularly patients bearing malignant tumors, in therapeutically effective doses. Suitable dosages will be apparent to the artisan in the therapeutic context, as is further described infra.

## Brief Description of the Drawings

Fig. 1 shows the elution profile of tumor necrosis factor from controlled pore glass.
Fig. 2 shows the elution profile of tumor necrosis factor from diethylaminoethyl cellulose.
Fig. 3 shows the elution profile of tumor necrosis factor from Fast protein liquid chromatography.
Fig. 4 shows the elution profile of tumor necrosis factor upon chromatofocusing.
Fig. 5 shows the molecular weight of tumor necrosis factor by SDS PAGE gel electrophoresis.
Fig. 6 shows the molecular weight of tumor necrosis factor upon HPLC elution.
Fig. 7 shows the elution profile of tumor necrosis factor from an HPLC C4 column.
Fig. 8 illustrates the separation of tumor necrosis factor trypsin digest fragments on HPLC.

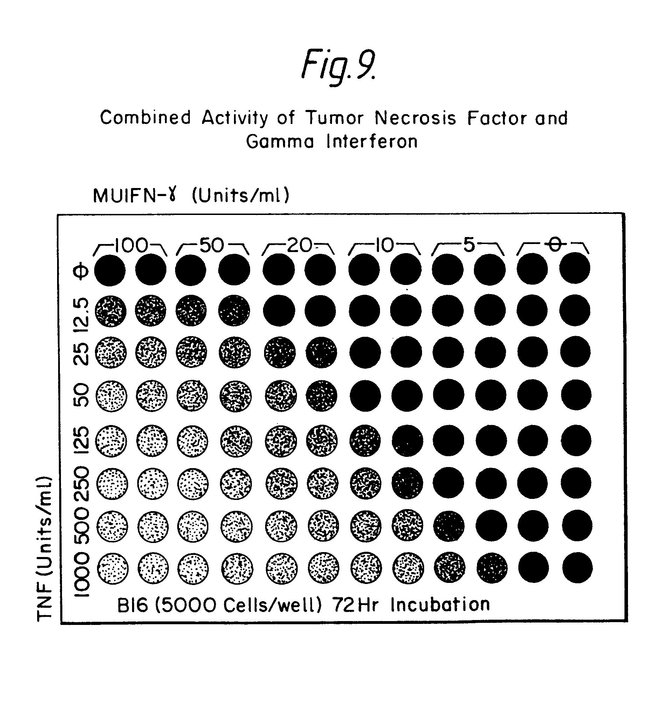

Fig. 9 illustrates the cytotoxic effect of gamma interferon and tumor necrosis factor mixtures.
Fig. 10 sets forth the nucleotide and amino acid sequence for pre human tumor necrosis factor, including the complete tumor necrosis factor secretory leader.
Fig. 11 shows the construction of a tumor necrosis factor expression vector.

## Detailed Description

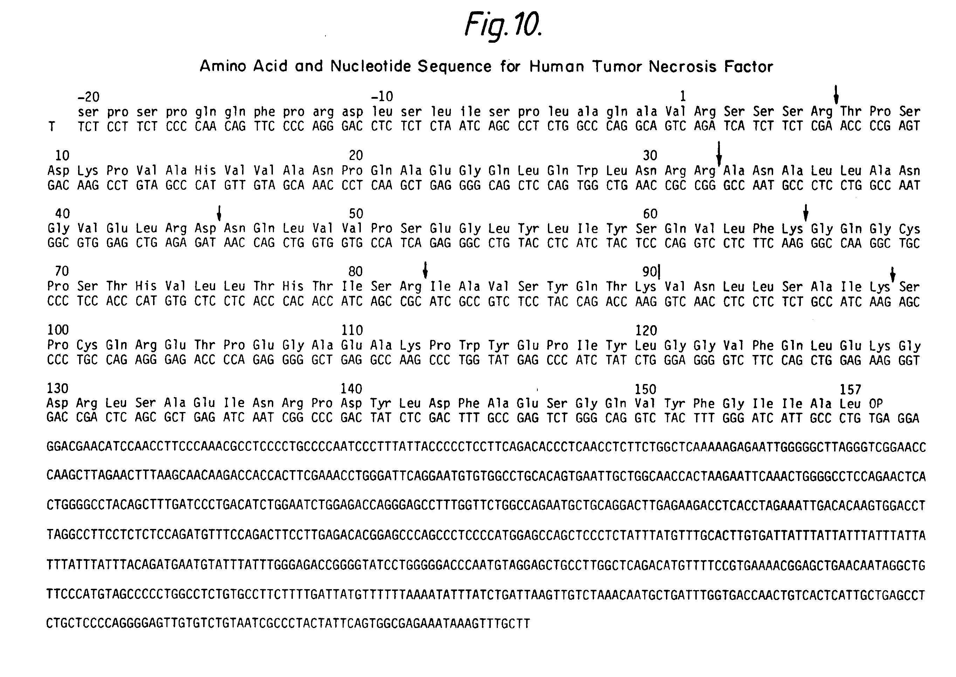

Tumor necrosis factor is defined for the purposes herein as a polypeptide other than lymphotoxin capable of preferential cytotoxic activity and having a region showing functional amino acid homology with the mature tumor necrosis factor amino acid sequence set forth in Fig. 10, a fragment thereof, or a derivative of such polypeptide or fragment.

Preferential cytotoxic activity is defined as the preferential destruction or growth inhibition of tumor cells when compared to normal cells under the same conditions. Preferential cytotoxic activity is detected by the effect of the polypeptide on tumor cells in vivo or in vitro as compared with normal cells or tissue. Cell lysis is generally the diagnostic indicia in vitro, while tumor necrosis is determined in in vivo experiments. However, cytotoxic activity may be manifest as cytostasis or antiproliferative activity. Suitable assay systems are well known. For example, the cell-lytic assay used to determine the specific activity of tumor necrosis factor described below is acceptable, as is the assay described in B. Aggarwal et al., in "Thymic Hormones and Lymphokines", 1983, ed. A. Goldstein, Spring Symposium on Health Sciences, George Washington University Medical Center (the A549 cell line referred to in this literature is available from the ATCC as CCL185).

The specific activity of TNF is cast in terms of target cell lysis, rather than cytostasis. One unit of tumor necrosis factor is defined as the amount required for 50 percent lysis of the target cells plated in each well in accord with Example 1. However, this is not meant to exclude other assays for measuring specific activity, e.g. methods based on target cell growth rate.

PreTNF is a species of tumor necrosis factor included within the foregoing definition of tumor necrosis factor. It is characterized by the presence in the molecule of a signal (or leader) polypeptide which serves to post-translationally direct the protein to a site inside or outside of the cell. Generally, the signal polypeptide (which will not have tumor necrotic activity in its own right) is proteolytically cleaved from a residual protein having tumor necrosis factor activity as part of the secretory process in which the protein is transported into the host cell periplasm or culture medium. The signal peptide may be microbial or mammalian (including the native, 76 residue presequence), but it preferably is a signal which is homologous to the host cell.

Native tumor necrosis factor from normal biological sources has a calculated molecular weight of about 17,000 on sodium dodecyl sulfate polyacrylamide gel electrophoresis (SDS-PAGE) as described infra, an isoelectric point of about 5.3, and susceptibility to trypsin hydrolysis at multiple sites. Native tumor necrosis factor which has been purified by reverse phase HPLC is trypsin hydrolyzed into at least nine fragments under the conditions described infra. The precise number of fragments into which tumor necrosis factor is hydrolyzed by trypsin will depend upon such factors as trypsin activity, tumor necrosis factor concentration and incubation parameters, including the ionic strength, pH, temperature and time of incubation.

Tumor necrosis factor does not appear to be a glycoprotein, as it is not retained on lectin affinity columns and analysis of the deduced amino acid sequence contains no likely glycosylation sites. Also, material produced in recombinant E. coli culture (which does not have the ability to glycosylate) co-migrates with natural TNF on SDS-PAGE.

The degree of amino acid sequence homology which brings a polypeptide within the scope of the definition of tumor necrosis factor herein will vary depending upon whether the homology between the candidate protein and tumor necrosis factor falls within or without the tumor necrosis factor regions responsible for cytotoxic activity; domains which are critical for cytotoxic activity should exhibit a high degree of homology in order to fall within the definition, while sequences not involved in maintaining tumor necrosis factor conformation or in effecting receptor binding may show comparatively low homology. In addition, critical domains may exhibit cytolytic activity and yet remain homologous as defined herein if residues containing functionally similar amino acid side chains are substituted. Functionally similar refers to dominant characteristics of the side chains such as basic, neutral or acid, or the presence or absence of steric bulk. However, tumor necrosis factor as defined herein specifically excludes lymphotoxin.

Generally a polypeptide defined as tumor necrosis factor will contain regions substantially homologous with the Fig. 10 protein or fragments thereof over a continuous block of about from 20 to 100 amino acid residues in particular the blocks encompassed by residues 35-66 and 110-133.

A most significant factor in establishing the identity of a polypeptide as tumor necrosis factor is the ability of antisera which are capable of substantially neutralizing the cytolytic activity of mature tumor necrosis factor as set forth in Fig. 10 to also substantially neutralize the cytolytic activity of the polypeptide in question. However it will be recognized that immunological identity and cytotoxic identity are not necessarily coextensive. A neutralizing antibody for the tumor necrosis factor of Fig. 10 may not bind a candidate protein because the neutralizing antibody happens to not be directed to specifically bind a site on tumor necrosis factor that is critical to its cytotoxic activity. Instead, the antibody may bind an Innocuous region and exert its neutralizing effect by steric hindrance. Therefore a candidate protein mutated in this innocuous region might no longer bind the neutralizing antibody, but it would nonetheless be tumor necrosis factor in terms of substantial homology and biological activity.

It is important to observe that characteristics such as molecular weight, isoelectric point and the like for the native or wild type mature human tumor necrosis factor of Fig. 10 obtained from peripheral lymphocyte or established cell line cultures are descriptive only for the native species of tumor necrosis factor. Tumor necrosis factor as contemplated by the foregoing definition will include other species which will not exhibit all of the characteristics of native tumor necrosis factor. While tumor necrosis factor as defined herein includes native tumor necrosis factor, other related cytotoxic polypeptides will fall within the definition. For example, TNF derivatives like the insertion mutants, deletion mutants, or fusion proteins described above will bring tumor necrosis factor outside of the molecular weight established for native human tumor necrosis factor (fusion proteins with mature tumor necrosis factor or preTNF itself as well as insertion mutants will have a greater molecular weight than native, mature tumor necrosis factor, while deletion mutants of native, mature tumor necrosis factor will have a lower molecular weight). Similarly, tumor necrosis factor may be engineered in order to reduce or eliminate susceptibility to hydrolysis by trypsin or other proteases. Finally, post-translational processing of human preTNF in cell lines derived from nonprimate mammals may produce microheterogeneity in the amino terminal region, so that valine will no longer be the amino terminal amino acid.

The amino acid sequence for human tumor necrosis factor deduced from its cDNA is described in Fig. 10. Mature or native tumor necrosis factor is represented by amino acid residues 1 to 157. Note that this sequence includes a 76 residue signal sequence which is believed to be removed during normal processing of the translated transcript to produce the mature protein. Trypsin hydrolysis sites are denoted by arrows.

Note that the language "capable" of cytotoxic activity or in vivo tumor necrosis means that the term tumor necrosis factor includes polypeptides which can be converted, as by enzymatic hydrolysis, from an inactive state analogous to a zymogen to a polypeptide fragment which exhibits the desired biological activity. Typically, inactive precursors will be fusion proteins in which mature tumor necrosis factor is linked by a peptide bond at its carboxyl terminus to a human protein or fragment thereof. The sequence at this peptide bond or nearby is selected so as to be susceptible to proteolytic hydrolysis to release tumor necrosis factor, either in vivo or, as part of a manufacturing protocol, in vitro. The tumor necrosis factor that is so generated then will exhibit the definitionally-required cytotoxic activity.

While tumor necrosis factor ordinarily is meant to mean human tumor necrosis factor, tumor necrosis factor from sources such as murine, porcine, equine or bovine is included within the definition of tumor necrosis factor so long as it otherwise meets the standards described above for homologous regions and cytotoxic activity. TNF is not species specific, e.g., human TNF is active on mouse tumors. Therefore, TNF from one species can be used in therapy of another.

Tumor necrosis factor also includes multimeric forms. Tumor necrosis factor spontaneously aggregates into multimers, usually dimers or higher multimers. Multimers are cytotoxic and accordingly are suitable for use in in vivo therapy. While it is desirable to express and recover tumor necrosis factor as a substantially homogeneous multimer or monomer, tumor necrosis factor may be used therapeutically as a mixture of different multimers.

Derivatives of tumor necrosis factor are included within the scope of the term tumor necrosis factor. Derivatives include amino acid sequence mutants, glycosylation variants and covalent or aggregative conjugates with other chemical moieties. Covalent derivatives are prepared by linkage of functionalities to groups which are found in the TNF amino acid side chains or at the N- or C-termini, by means known in the art. These derivatives may, for example, include: aliphatic esters or amides of the carboxyl terminus or residues containing carboxyl side chains, e.g., asp10; O-acyl derivatives of hydroxyl group-containing residues such as ser52, ser3, ser4 or ser5; N-acyl derivatives of the amino terminal amino acid or amino-group containing residues, e.g. lysine or arginine; and derivatives of cys101 and cys69. The acyl group is selected from the group of alkyl-moieties (including C3 to C10 normal alkyl), thereby forming alkanoyl species, and carbocyclic or heterocyclic compounds, thereby forming aroyl species. The reactive groups preferably are difunctional compounds known per se for use in cross-linking proteins to insoluble matrices through reactive side groups. Preferred derivatization sites are at cysteine and histidine residues.

Covalent or aggregative derivatives are useful as reagents in immunoassay or for affinity purification procedures. For example, tumor necrosis factor is insolubilized by covalent bonding to cyanogen bromide-activated Sepharose by methods known per se or adsorbed to polyolefin surfaces (with or without glutaraldehyde cross-linking) for use in the assay or purification of anti-TNF antibodies or cell surface receptors. Tumor necrosis factor also is labelled with a detectable group, e.g., radioiodinated by the chloramine T procedure, covalently bound to rare earth chelates or conjugated to another fluorescent moiety for use in diagnostic assays, especially for diagnosis of TNF levels in biological samples by competitive-type immunoassays. Such derivatives may fall outside of the TNF definition above because it is not necessary that they show cytotoxic activity, only cross-reactivity with anti-TNF.

Mutant tumor necrosis factor derivatives include the predetermined, i.e. site specific, mutations of TNF or its fragments. The objective of mutagenesis is to construct DNA that encodes tumor necrosis factor as defined above, i.e., tumor necrosis factor which exhibits cytotoxic activity towards tumor cells in vitro or causes tumor necrosis in vivo, and which retains residual homology with the Fig. 10 sequence, but which also exhibits improved properties and activity. Mutant tumor necrosis factor is defined as a polypeptide otherwise falling within the homology definition for tumor necrosis factor set forth herein but which has an amino acid sequence different from that of tumor necrosis factor whether by way of deletion, substitution or insertion. For example, the lysine or arginine residues of tumor necrosis factor (arginine 2, 6, 82, 44 and 131 and lysine 98, 90, and 65,) may be mutated to histidine or another amino acid residue which does not render the protein proteolytically labile. Similarly, cysteine 101 could be replaced by other residues and cross-linked chemically in order to confer oxidative stability. It is not necessary that mutants meet the activity requirements for tumor necrosis factor, for even biologically inactive mutants will be useful upon labelling or immobilization as reagents in immunoassays. However, in this case the mutants will retain at least one epitopic site which is cross-reactive with antibody to tumor necrosis factor.

The regions of the tumor necrosis molecule within residues 35 to 66 and 110 to 133 inclusive show substantial homology (50 percent) with lymphotoxin. The hydrophobic carboxy-termini (tumor necrosis factor residues 150 to 157) of the two molecules also are significantly conserved. Since both proteins demonstrate cytotoxic activity or in vivo tumor necrosis these regions are believed to be important in the shared activity of lymphotoxin and tumor necrosis factor. As such, the residues in these regions are preferred for mutagenesis having the objective of directly affecting the activity of tumor necrosis factor on a given cell. The relatively unconserved region at tumor necrosis factor residues 67-109 may function to correctly position the two surrounding relatively homologous regions in a conformation essential for cytotoxic activity. Such positioning, which could be achieved by a Cys69-Cys101 disulfide bond in tumor necrosis factor, might be similarly accomplished by the corresponding region of lymphotoxin and could account for differences in specificity and activity between the two molecules. In this connection, since these residues are postulated to represent the active core of tumor necrosis factor they may be synthesized chemically or by deletion mutagenesis as truncated, short-length polypeptides having tumor necrosis factor activity.

While the mutation site is predetermined, it is unnecessary that the mutation per se be predetermined. For example, in order to optimize the performance of the position 131 mutants random mutagenesis may be conducted at the codon for arginine 131 and the expressed tumor necrosis factor mutants screened for the optimal combination of cytotoxic activity and protease resistance. Techniques for making substitution mutations at predetermined sites in DNA having a known sequence is well known, for example M13 primer mutagenesis.

Tumor necrosis factor mutagenesis is conducted by making amino acid insertions, usually on the order of about from 1 to 10 amino acid residues, or deletions of about from 1 to 30 residues. Substitutions, deletions, insertions or any subcombination may be combined to arrive at a final construct. Insertions include amino or carboxyl-terminal fusions, e.g. a hydrophobic extension added to the carboxyl terminus. Preferably, however, only substitution mutagenesis is conducted. Obviously, the mutations in the encoding DNA must not place the sequence out of reading frame and preferably will not create complementary regions that could produce secondary mRNA structure.

Not all mutations in the DNA which encode the tumor necrosis factor will be expressed in the final secreted product. For example, a major class of DNA substitution mutations are those in which a different secretory leader or signal has been substituted for the native human secretory leader, either by deletions within the leader sequence or by substitutions, wherein most or all of the native leader is exchanged for a leader more likely to be recognized by the intended host. For example, in constructing a procaryotic expression vector the human secretory leader is deleted in favor of the bacterial alkaline phosphatase or heat stable enterotoxin II leaders, and for yeast the leader is substituted in favor of the yeast invertase, alpha factor or acid phosphatase leaders. However, the human secretory leader may be recognized by hosts other than human cell lines, most likely in cell culture of higher eukaryotic cells. When the secretory leader is "recognized" by the host, the fusion protein consisting of tumor necrosis factor and the leader ordinarily is cleaved at the leader-tumor necrosis factor peptide bond in the events that lead to secretion of the tumor necrosis factor. Thus, even though a mutant preTNF DNA is used to transform the host, and mutant preTNF is synthesized as intermediate, the resulting tumor necrosis factor ordinarily is native, mature tumor necrosis factor.

Another major class of DNA mutants that are not expressed as tumor necrosis factor derivatives are nucleotide substitutions made to enhance expression, primarily to avoid amino terminal loops in the transcribed mRNA (see copending U.S.S.N. 303,687, incorporated by reference) or to provide codons that are more readily transcribed by the selected host, e.g. the well-known E. coli preference codons for E. coli expression.

Substantially homogeneous tumor necrosis factor means tumor necrosis factor which is substantially free of other proteins native to the source from which the tumor necrosis factor was isolated. This means that homogeneous tumor necrosis factor is substantially free of blood plasma proteins such as albumin, fibrinogen, serine proteases, $\alpha$-globulins, non-tumor necrosis factor cytotoxic polypeptides such as lymphotoxin or interferons, or other proteins of the cell or organism which serves as the synthetic origin of the tumor necrosis factor, including whole cells and particulate cell debris. However, homogeneous tumor necrosis factor may include such substances as the stabilizers and excipients described below, predetermined amounts of proteins from the cell or organism that serves as the synthetic origin, proteins from other than the tumor necrosis factor source cells or organisms, and synthetic polypeptides such as poly-L-lysine.

Recombinant tumor necrosis factor which is expressed in an allogeneic, e.g. bacterial, host cell of course will be expressed completely free of gene source proteins.

Tumor necrosis factor is preferably synthesized in cultures of recombinant organisms. Neither peripheral blood lymphocytes (PBLs) nor cell lines are desirable. It is difficult in practice to obtain PBLs of one class which are free of contamination by cells of other classes, e.g. to obtain macrophages free of B or T cells. Such contamination will render the separation procedure applied to the products of such cells more difficult because of other potential cytotoxic factors and proteins released by the contaminant cells. Furthermore, tumor necrosis factor obtained from nonrecombinant culture is expensive and will consist solely of native tumor necrosis factor, such cultures thereby lacking in the flexibility of recombinant culture to improve upon the characteristics of tumor necrosis factor.

DNA which encodes tumor necrosis factor is obtained by chemical synthesis, by screening reverse transcripts of mRNA from PBL or cell line cultures, or by screening genomic libraries from any cell. Suitable cell line cultures comprise monocytic cell lines such as promyelocytic cell lines designated "HL-60" in the art (one of which is available from the ATCC as CCL 240) or the histiocytic lymphoma cell line U 937 (ATCC CRL 1593). These and other cell lines are induced to express and secrete tumor necrosis factor by exposure of the cells to chemical or physical agents known in the art, generally tumorigenic or mitogenic agents. Tumor necrosis factor can be induced effectively in certain monocytic cell lines only with PMA; otherwise conventional agents such lipopolysaccharide, staphylococcal enterotoxin B, or thymosin $\alpha$-1 were not as effective as PMA at inducing tumor necrosis factor in these cell lines. Since a variable amount of screening is required to locate a cell line expressing tumor necrosis factor (and therefore containing the desired mRNA) it may be more efficient to simply synthesize the gene. Synthesis is advantageous because unique restriction sites can be introduced (thereby facilitating the use of the gene in vectors containing restriction sites otherwise present in the native sequence) and steps can be taken to enhance translational efficiency, as discussed below.

This DNA is covalently labelled with a detectable substance such as a fluorescent group, a radioactive atom or a chemiluminescent group by methods known per se. It is then used in conventional hybridization assays. Such assays are employed in identifying TNF vectors and transformants as described in the Examples infra, or for in vitro diagnosis such as detection of TNF mRNA in blood cells.

The mRNA for TNF, surprisingly, is relatively rare even in induced HL-60 cells, perhaps due to instability in the messenger resulting from unknown causes. Furthermore, the time course of appearance of TNF mRNA after cell induction is important. TNF mRNA appears in the cells for only a brief period at about 4 hours post-induction. In this respect its appearance is distinct from that of lymphotoxin, which appears at about 12 hours post-induction. This makes the cDNA easy to overlook were one not apprised as to what to look for. However, once its presence is appreciated and completely complementary DNA made available, as is enabled by the disclosures herein, it is routine to screen cDNA libraries of induced HL-60 or PBLs for tumor necrosis factor cDNA using probes having sequences of such DNA. The two HL-60 phage libraries screened in the Examples contain a relatively consistent number of positive plaques, so it is clear that routine hybridization assays will identify phage containing the desired cDNA.

Tumor necrosis factor-synthesizing cells of an HL-60 cell line initially are cultured in conventional fashion until reaching a density of about 8 - 12 x $10^5$ cells/ml. The cells are removed frown the culture, washed, transferred to serum-free medium and grown in medium containing PMA. The culture then is continued until the desired concentration of tumor necrosis factor has accumulated in the culture medium, ordinarily about 400 tumor necrosis factor units/ml. Thereafter the culture supernatant is preferably clarified by centrifugation or other means of separating cell debris from the soluble components. Centrifugation should be carried out at low speed so as to move only suspended particles. The supernatant is then purified as described infra.

Alternatively, and preferably, tumor necrosis factor is synthesized in host cells transformed with vectors containing DNA encoding tumor necrosis factor. A vector is a replicable DNA construct. Vectors are used herein either to amplify DNA encoding tumor necrosis factor and/or to express DNA which encodes tumor necrosis factor. An expression vector is a replicable DNA construct in which a DNA sequence encoding tumor necrosis factor is operably linked to suitable control sequences capable of effecting the expression of tumor necrosis factor in a suitable host. Such control sequences include a transcriptional promoter, an optional operator sequence to control transcription, a sequence encoding suitable mRNA ribosomal binding sites, and sequences which control termination of transcription and translation.

Vectors comprise plasmids, viruses (including phage), and integratable DNA fragments (i.e., integrable into the host genome by recombination). Once it has transformed a suitable host, the vector replicates and functions independently of the host genome, or may, in some instances, integrate into the genome itself. In the present specification, "vector" is generic to "plasmid", but plasmids are the most commonly used form of vector at present. However, all other forms of vectors which serve an equivalent function and which are, or become, known in the art are suitable for use herein. Suitable vectors will contain replicon and control sequences which are derived from species compatible with the intended expression host. Transformed host cells are cells which have been transformed or transfected with tumor necrosis factor vectors constructed using recombinant DNA techniques. Transformed host cells ordinarily express tumor necrosis factor. The expressed tumor necrosis factor will be deposited intracellularly or secreted into either the periplasmic space or the culture supernatant, depending upon the host cell selected.

DNA regions are operably linked when they are functionally related to each other. For example, DNA for a presequence or secretory leader is operably linked to DNA for a polypeptide if it is expressed as a preprotein which participates in the secretion of the polypeptide; a promoter is operably linked to a coding sequence if it controls the transcription of the sequence; or a ribosome binding site is operably linked to a coding sequence if it is positioned so as to permit translation. Generally, operably linked means contiguous and, in the case of secretory leaders, contiguous and in reading phase.

Suitable host cells are prokaryotes, yeast or higher eukaryotic cells. Prokaryotes include gram negative or gram positive organisms, for example E. coli or Bacilli. Higher eukaryotic cells include established cell lines of mammalian origin as described below. A preferred host cell is the phage resistant E. coli W3110 (ATCC 27,325) strain described in the Examples, although other prokaryotes such as E. coli B, E. coli X1776 (ATCC 31,537), E. coli 294 (ATCC 31,446), pseudomonas species, or Serratia Marcesans are suitable.

Prokaryotic host-vector systems are preferred for the expression of tumor necrosis factor. While the tumor necrosis factor molecule contains two cysteine residues, thereby implying a modest potential requirement for post-translational processing to form a potential disulfide bond, E. coli for example expresses biologically active tumor necrosis factor. A plethora of suitable microbial vectors are available. Generally, a microbial vector will contain an origin of replication recognized by the intended host, a promoter which will function in the host and a phenotypic selection gene, for example a gene encoding proteins conferring antibiotic resistance or supplying an auxotrophic requirement. Similar constructs will be manufactured for other hosts. E. coli is typically transformed using pBR322, a plasmid derived from an E. coli species (Bolivar, et al., 1977, "Gene" 2: 95). pBR322 contains genes for ampicillin and tetracycline resistance and thus provides easy means for identifying transformed cells.

Vectors must contain a promoter which is recognized by the host organism. This is generally a promoter homologous to the intended host. Promoters most commonly used in recombinant DNA construction include the β-lactamase (penicillinase) and lactose promoter systems (Chang et al., 1978, "Nature", 275: 615; and Goeddel et al., 1979, "Nature" 281: 544), a tryptophan (trp) promoter system (Goeddel et al., 1980, "Nucleic Acids Res." 8: 4057 and EPO App. Publ. No. 36,776) and the tac promoter [H. De Boer et al., "Proc. Nat'l. Acad. Sci. U.S.A." 80: 21-25 (1983)]. While these are the most commonly used, other known microbial promoters are suitable. Details concerning their nucleotide sequences have been published, enabling a skilled worker operably to ligate them to DNA encoding tumor necrosis factor in plasmid vectors (Siebenlist et al., 1980, "Cell" 20: 269) and the DNA encoding tumor necrosis factor. At the present time the preferred vector is a pBR322 derivative containing the E. coli alkaline phosphatase promoter with the trp Shine-Dalgarno sequence. The promoter and Shine-Dalgarno sequence are operably linked to the DNA encoding the TNF, i.e., they are positioned so as to promote transcription of TNF mRNA from the DNA.

In addition to prokaryates, eukaryotic microbes such as yeast cultures are transformed with tumor necrosis factor-encoding vectors. Saccharomyces cerevisiae, or common baker's yeast is the most commonly used among lower eukaryotic host microorganisms, although a number of other strains are commonly available. Yeast vectors generally will contain an origin of replication from the 2 micron yeast plasmid or an autonomously replicating sequence (ARS), a promoter, TNF, sequences for polyadenylation and transcription termination and a selection gene. A suitable plasmid for tumor necrosis factor expression in yeast is YRp7, (Stinchcomb et al., 1979, "Nature", 282: 39; Kingsman et al., 1979, "Gene", 7: 141; Tschemper et al., 1980, "Gene", 10: 157). This plasmid already contains the trp1 gene which provides a selection marker for a mutant strain of yeast lacking the ability to grow in tryptophan, for example ATCC No. 44076 or PEP4-1 (Jones, 1977, "Genetics", 85: 12). The presence of the trp1 lesion in the yeast host cell genome then provides an effective environment for detecting transformation by growth in the absence of tryptophan.

Suitable promoting sequences in yeast vectors include the promoters for metallothionein, 3-phosphoglycerate kinase (Hitzeman et al., 1980, "J. Biol. Chem.", 255: 2073) or other glycolytic enzymes (Hess et al., 1968, "J. Adv. Enzyme Reg.", 7: 149; and Holland et al., 1978, "Biochemistry", 17: 4900), such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase. Suitable vectors and promoters for use in yeast expression are further described in R. Hitzeman et al., EPO Publn. No. 73,657.

Other promoters, which have the additional advantage of transcription controlled by growth conditions, are the promoter regions for alcohol dehydrogenase 2, isocytochrome C, acid phosphatase, degradative enzymes associated with nitrogen metabolism, and the aforementioned metallothionein and glyceraldehyde-3- phosphate dehydrogenase, as well as enzymes responsible for maltose and galactose utilization. In constructing suitable expression plasmids, the termination sequences associated with these genes are also ligated into the expression vector 3' of the tumor necrosis factor coding sequences to provide polyadenylation of the mRNA and termination.

In addition to microorganisms, cultures of cells derived from multicellular organisms may also be used as hosts. This, however, is not preferred because of the excellent results obtained thus far with TNF expressing microbes. In principal, any higher eukaryotic cell culture is workable, whether from vertebrate or invertebrate culture. However, interest has been greatest in vertebrate cells, and propagation of vertebrate cells in culture (tissue culture) has become a routine procedure in recent years [Tissue Culture, Academic Press, Kruse and Patterson, editors (1973)]. Examples of useful host cell lines are VERO and HeLa cells, Chinese hamster ovary (CHO) cell lines, and WI38, BHK, COS-7 and MDCK cell lines. Expression vectors for such cells ordinarily include (if necessary) an origin of replication, a promoter located upstream from the gene to be expressed, along with a ribosome binding site, RNA splice site (if intron-containing genomic DNA is used), a polyadenylation site, and a transcriptional termination sequence.

The transcriptional and translational control sequences in expression vectors to be used in transforming vertebrate cells are often provided by viral sources. For example, commonly used promoters are derived from polyoma, Adenovirus 2, and most preferably Simian Virus 40 (SV40). The early and late promoters are particularly useful because both are obtained easily from the virus as a fragment which also contains the SV40 viral origin of replication (Fiers et al., 1978, "Nature", 273: 113). Smaller or larger SV40 fragments may also be used, provided the approximately 250 bp sequence extending from the Hind III site toward the Bg1 I site located in the viral origin of replication is included. Further, it is also possible, and often desirable, to utilize human genomic promoter, control and/or signal sequences normally associated with tumor necrosis factor, provided such control sequences are compatible with the host cell systems.

An origin of replication may be provided either by construction of the vector to include an exogenous origin, such as may be derived from SV40 or other viral (e.g. Polyoma, Adenovirus, VSV, or BPV) source, or may be provided by the host cell chromosomal replication mechanism. If the vector is integrated into the host cell chromosome, the latter is often sufficient.

In selecting a preferred host mammalian cell for transfection by vectors which comprise DNA sequences encoding both tumor necrosis factor and dihydrofolate reductase (DHFR), it is appropriate to select the host according to the type of DHFR protein employed. If wild type DHFR protein is employed, it is preferable to select a host cell which is deficient in DHFR thus permitting the use of the DHFR coding sequence as a marker for successful transfection in selective medium which lacks hypoxanthine, glycine, and thymidine. An appropriate host cell in this case is the Chinese hamster ovary (CHO) cell line deficient in DHFR activity, prepared and propagated as described by Urlaub and Chasin, 1980, "Proc. Natl. Acad. Sci." (USA) 77: 4216.

On the other hand, if DNA encoding DHFR protein with low binding affinity for methotrexate (MTX) is used as the controlling sequence, it is not necessary to use DHFR resistant cells. Because the mutant DHFR is resistant to MTX, MTX containing media can be used as a means of selection provided that the host cells are themselves MTX sensitive. Most eukaryotic cells which are capable of absorbing MTX appear to be methotrexate sensitive. One such useful cell line is a CHO line, CHO-K1 (ATCC No. CCL 61).

Tumor necrosis factor initially is recovered from cultures. Transformed nonsecreting cells are lysed by sonication or other acceptable method and debris separated by centrifugation, while the supernatants from secreting cells (such as induced cell lines) are simply separated from the cells by centrifugation. Then any one or more of the following steps may be used, or other methods entirely may be substituted. The following method was used to purify tumor necrosis factor to a degree sufficient for sequencing. This is not necessarily coextensive with the purification required for a therapeutic product.

As an initial purification step, tumor necrosis factor is adsorbed onto a hydrophobic substance from the lysed culture or supernatant culture medium. The hydrophobic substance preferably is a nongelatinous hydrophobic surface such as a silicate or polyolefin, although alkyl Sepharose also is suitable. The prefered embodiment is controlled pore glass. A ratio of about 1 volume of controlled pore glass is mixed with 50 volumes of supernatant and the adsorption allowed to proceed at about 4°C without agitation over a period of about 30 minutes to 2 hours, preferably about 1 hour, under slightly alkaline conditions. The adsorbent generally should thereafter be washed with a suitable buffer to remove entrapped contaminant proteins.

The adsorbed tumor necrosis factor is eluted from the hydrophobic substance by altering the solvation properties of the surrounding medium. The elution can be accomplished by passing a solution buffered at approximately pH 7 to 8.5, preferably around 8, containing 1M salt and an effective amount of an aqueous solution of a water miscible organic polyol, such as, for example, ethylene glycol or glycerin, ordinarily ethylene glycol in the range of 10-30 percent v/v, preferably around 20 percent v/v. Of course the optimum conditions will depend upon the polyol which is used. The tumor necrosis factor-containing elution fractions are detected by in vitro assay as described below or by other suitable assay. The purification and yield of this step from monocytic cell culture, as well as subsequent steps, are shown below in Table I.

Further purification is obtained by adsorption of tumor necrosis factor onto a tertiary or quaternary amino anion exchange resin. The preferred resins for this purpose are hydrophilic matrix resins such as cross-linked polystyrene, dextran or cellulose substituted with alkyl tertiary or quaternary amino groups. Commercial products of this type are available as DEAE cellulose, QAE Sephadex or under the trade name Mono Q (in each instance where ethyl is the alkyl substituent in each of these products). Best results are achieved with the fast protein liquid chromatography system described by J. Richey, October 1982, "American Laboratory" using the macroporous substantially uniform particles of Ugelstad et al., 1933, "Nature" 303: 95-96. This system has enabled the purification of tumor necrosis factor to a high level.

Purification to substantial homogeneity is achieved only upon further separation on SDS PAG electrophoresis or C4-reverse phase high pressure liquid chromatography (HPLC) as described in the Examples below. This product, however, is not desirable for therapy because it has lost substantial activity upon exposure to SDA or HPLC organic solvent. Protein concentration was determined by the method of M. Bradford, 1976, "Anal. Biochem." 72:248-254. During the final stages of purification, the protein concentration was estimated by amino acid composition and also by amino acid sequence.

Tumor necrosis factor is prepared for administration by mixing tumor necrosis factor having the desired degree of purity with physiologically acceptable carriers, i.e., carriers which are nontoxic to recipients at the dosages and concentrations employed. Ordinarily, this will entail combining the tumor necrosis factor with buffers, antioxidants such as ascorbic acid, low molecular weight (less than about 10 residues) polypeptides, proteins, amino acids, carbohydrates including glucose or dextrins, chelating agents

Table I

Purification of Human Tumor Necrosis Factor from HL-60 Cell Culture Medium

| Purification Step | Final Volumes (ml) | Total Protein (mg) | Cytolytic Activity (units) | Relative Specific Activity (units/mg) | Purification | Recovery (Percent) |
|---|---|---|---|---|---|---|
| Starting Material | 58,000 | 1,964 | $14.2 \times 10^6$ | $0.007 \times 10^6$ | — | — |
| Controlled Pore Glass Chromatography | 1,080 | 88.9 | $11.1 \times 10^6$ | $0.12 \times 10^6$ | 17 | 78.5 |
| DEAE-Cellulose Chromatography | 285 | 9.05 | $8.9 \times 10^6$ | $0.98 \times 10^6$ | 140 | 62.7 |
| Mono Q – Fast Protein Liquid Chromatography | 75 | 0.44 | $6.9 \times 10^6$ | $15.68 \times 10^6$ | 2,240 | 48.6 |
| Preparative SDS PAG Electrophoresis or C4-Reverse phase-HPLC | 6 | 0.028 | $2.71 \times 10^6$* | $96.79 \times 10^6$* | 13,387* | 19.1* |

* Corrected for partial destruction of tumor necrosis factor activity caused by SDS, or by TFA and propanol.

such as EDTA, and other stabilizers and excipients. The carrier should be formulated to stabilize the tumor necrosis factor as a dimer and/or, preferably, a trimer. this is accomplished by avoiding salts or detergents in concentrations that dissociate tumor necrosis factor into monomers. Alternatively, conditions that aggregate tumor necrosis factor into higher multimers should be avoided. Generally a nonionic surfactant such as Tween 20 is employed to exhibit excessive aggregation during purification as well as lyophilization or aqueous storage. Tumor necrosis factor to be used for therapeutic administration must be sterile. This is readily accomplished by filtration through sterile filtration membranes. Tumor necrosis factor ordinarily will be stored in lyophilized form.

Tumor necrosis factor optionally is combined with other antineoplastic agents such as chemotherapeutic antibiotics (actinomycin-D, adriamycin, aclacinomycin A), or with agents to augment or stimulate the immune response, for example immunoglobulins such as gamma globulin, including immunoglobulins having affinity for the cell surface antigens of neoplasms. In addition, since interferons act synergistically with tumor necrosis factor in cell lysis assays, alpha, beta or gamma interferon is desirably combined with tumor necrosis factor compositions or tumor necrosis factor and lymphotoxin-containing compositions. A typical formulation comprises tumor necrosis factor and gamma interferon in a unit activity proportion of about from 0.1:1 to 200:1, ordinarily 10 to 1, and may contain lymphotoxin in place of a proportion of tumor necrosis factor. These proportions, of course, are subject to modification as required by therapeutic experience.

Tumor necrosis factor compositions are administered to tumor-bearing animals. The route of administration is in accord with known methods, e.g. the intravenous, intraperitoneal, intramuscular, intralesional infusion or injection of sterile tumor necrosis factor solutions, or by timed release systems as noted below. Tumor necrosis factor is administered intralesionally, i.e., by direct injection into solid tumors. In the case of disseminated tumors such as leukemia, administration is preferably intravenous or into the lymphatic system. Tumors of the abdominal organs such as ovarian cancer are advantageously treated by intraperitoneal infusion using peritoneal dialysis hardware and peritoneal-compatible solutions. Ordinarily, however, tumor necrosis factor is administered continuously by infusion although bolus injection is acceptable.

Tumor necrosis factor desirably is administered from an implantable timed-release article. Examples of suitable system for proteins having the molecular weight of tumor necrosis factor dimers or trimers include copolymers of L-glutamic acid and gamma ethyl-L-glutamate (U. Sidman et al., 1983, "Biopolymers" 22 (1): 547-556), poly (2-hydroxyethyl-methacrylate) (R. Langer et al., 1981, "J. Biomed. Mater. Res." 15: 167-277 and R. Langer, 1982, "Chem. Tech." 12: 98-105) or ethylene vinyl acetate (R Langer et al.,Id.). This article is implanted at surgical sites from which tumors have been excised. Alternatively, tumor necrosis factor is encapsulated in semipermeable microcapsules or liposomes for injection into the tumor. This mode of administration is particularly useful for surgically inexcisable tumors, e.g. brain tumors.

The amount of tumor necrosis factor that is administered will depend, for example, upon the route of administration, the tumor in question and the condition of the patient. Intralesional injections will require less tumor necrosis factor on a body weight basis than will intravenous infusion, while some tumor types, e.g., solid tumors appear to be more resistant to tumor necrosis factor than others, e.g. leukemic. Accordingly, it will be necessary for the therapist to titer the dosage and modify the route of administration as required to obtain optimal cytotoxic activity towards the target tumor, as can be determined for example by biopsy of the tumor or diagnostic assays for putative cancer markers such as carcinoembryonic antigen, in view of any recombinant toxicity encountered at elevated dosage. Ordinarily, tumor necrosis factor dosages in mice up to about 120 micrograms/kg body weight/day by intravenous administration have been found to be substantially nontoxic and efficacious in vivo.

Tumor necrosis factor is not believed to be species specific in its cytotoxic activity, so that tumor necrosis factor other than human tumor necrosis factor, e.g. from bovine or porcine sources, might be employed in the therapy of human tumors. However, it is desired to use tumor necrosis factor from the species being treated in order to avoid the potential generation of autoantibodies.

In order to simplify the Examples certain frequently occurring methods will be referenced by shorthand phrases.

Plasmids are designated by a low case p preceded and/or followed by capital letters and/or numbers. The starting plasmids herein are commercially available, are publically available on an unrestricted basis, or can be constructed from such available plasmids in accord with published procedures. In addition, other equivalent plasmids are known in the art and will be apparent to the ordinary artisan.

"Digestion" of DNA refers to catalytic cleavage of the DNA with an enzyme that acts only at certain locations in the DNA. Such enzymes are called restriction enzymes, and the sites for which each is specific is called a restriction site. "Partial" digestion refers to incomplete digestion by a restriction enzyme, i.e., conditions are chosen that result in cleavage of some but not all of the sites for a given restriction endonuclease in a DNA substrate. The various restriction enzymes used herein are commercially available and their reaction conditions, cofactors and other requirements as established by the enzyme suppliers were used. Restriction enzymes commonly are designated by abbreviations composed of a capital letter followed by other letters and then, generally, a number representing the microorganism from which each restriction enzyme originally was obtained. In general, about 1 $\mu$g of plasmid or DNA fragment is used with about 1 unit of enzyme in about 20 $\mu$l of buffer solution. Appropriate buffers and substrate amounts for particular restriction enzymes are specified by the manufacturer. Incubation times of about 1 hour at 37°C are ordinarily used, but may vary in accordance with the supplier's instructions. After incubation, protein is removed by extraction with phenol and chloroform, and the digested nucleic acid is recovered from the aqueous fraction by precipitation with ethanol. Digestion with a restriction enzyme infrequently is followed with bacterial alkaline phosphatase hydrolysis of the terminal 5' phosphates to prevent the two restriction cleaved ends of a DNA fragment from "circularizing" or forming a closed loop that would impede insertion of another DNA fragment at the restriction site. Unless otherwise stated, digestion of plasmids is not followed by 5' terminal dephosphorylation. Procedures and reagents for dephosphorylation are conventional (T. Maniatis et al., 1982, Molecular Cloning pp. 133-134).

"Recovery" or "isolation" of a given fragment of DNA from a restriction digest means separation of the digest on polyacrylamide gel electrophoresis, identification of the fragment of interest by comparison of its mobility versus that of marker DNA fragments of known molecular weight, removal of the gel section containing the desired fragment, and separation of the gel from DNA. This procedure is known generally. For example, see R. Lawn et al., 1981, "Nucleic Acids Res." 9: 6103-6114, and D. Goeddel et al., 1980, "Nucleic Acids Res." 8: 4057.

"Southern Analysis" is a method by which the presence of DNA sequences in a digest or DNA-containing composition is confirmed by hybridization to a known, labelled oligonucleotide or DNA fragment. For the purposes herein, unless otherwise provided, Southern analysis shall mean separation of digests on 1 percent agarose, denaturation and transfer to nitrocellulose by the method of E. Southern, 1975, "J. Mol. Biol." 98: 503-517, and hybridization as described by T. Maniatis et al., 1978, "Cell" 15: 687-701.

"Transformation" means introducing DNA into an organism so that the DNA is replicable, either as an extrachromosomal element or chromosomal integrant. Unless otherwise provided, the method used herein for transformation of E. coli is the CaCl$_2$ method of Mandel et al., 1970, "J. Mol. Biol." 53: 154.

"Ligation" refers to the process of forming phosphodiester bonds between two double stranded nucleic acid fragments (T. Maniatis et al., Id., p. 146). Unless otherwise provided, ligation may be accomplished using known buffers and conditions with 10 units of T4 DNA ligase ("ligase") per 0.5 $\mu$g of approximately equimolar amounts of the DNA fragments to be ligated.

"Preparation" of DNA from transformants means isolating plasmid DNA from microbial culture. Unless otherwise provided, the alkaline/SDS method of Maniatis et al., Id. p. 90., may be used.

"Oligonucleotides" are short length single or double stranded polydeoxynucleotides which are chemically synthesized by known methods and then purified on polyacrylamide gels.

All literature citations are expressly incorporated by reference.

Example 1

Assays

The specific activity of tumor necrosis factor was determined by a previously described modified cell lytic assay (B. Spofford, 1974, "J. Immun." 112: 2111). Mouse L-929 fibroblast cells (ATCC CCL-929) were grown in 96 well flat bottomed trays (3040; Falcon Plastics, Oxnard, CA) at 30,000 cells (vol 0.1 ml) per well in the presence of 1 $\mu$g/ml of actinomycin D and a serially diluted test sample (0.125 ml). The cells were incubated in a humidified atmosphere at 37°C with 5 percent CO$_2$. The test sample was removed after 18 h, the plates were washed and cell lysis was detected by staining the plates with 0.5 percent solution of crystal violet in methanol:water (1:4)(v/v). The endpoint on the microtiter plates was determined by a Microelisa autoreader (Dynatech) set for absorption at 450 nm and transmission at 570 nm. The cells exposed to culture medium alone were set at 0 percent lysis and those exposed to 3M guanidine hydrochloride solution provided an end point for 100 percent lysis. One unit of tumor necrosis factor is defined as the amount of tumor necrosis factor (when assayed in the 0.125 ml volume) which is required for 50 percent cell lysis.

Tumor necrosis factor also was tested in an in vivo tumor necrosis assay. Briefly, this assay was carried out by growing Meth A Sarcoma cells (5 x 10$^5$ cells) in CB6F$_1$ female mice (BALB/c x C57BL/6)F$_1$ for 7-10 days and then injecting intratumorly with a sample of tumor necrosis factor. After 24 hrs, mice were sacrificed by cervical dislocation, tumors were removed and the necrosis was scored histologically as previously described in E. Carswell et al., 1975, "Proc. Nat. Acad. Sci." 72:3666-3670.

Example 2

Use of PBLs or a Monocytic Cell Line to Synthesize Tumor Necrosis Factor

An HL-60 human promyelocytic cell line seed culture having a cell density of $1 \times 10^5$ cells/ml was grown in 2-liter roller bottles (890 cm$^2$) using 500 ml of RPMI 1640 medium (Irvine Scientific, Santa Ana, CA) containing 10 mM HEPES, 0.05 mM $\beta$-mercaptoethanol, 100 units/ml penicillin, 100 $\mu$g/ml streptomycin and 10 percent fetal calf serum. After three days at 37°C, when the culture reached a cell density of 8-12 x $10^5$ cells/ml, cells were harvested by centrifugation at 1000g for 10 min., washed twice with serum free RPMI 1640 medium and transferred into the same medium as described above without serum at a cell density of 15-20 x $10^5$ cells/ml. Cells were grown in 2-liter roller bottles in the presence of 10 ng/ml PMA. After 16-24 h, the cells were removed by filtration through a 3 $\mu$m Sealkleen filter (Pall Trinity Micro Corp. Cortland, NY). The clear filtrate was assayed for tumor necrosis factor activity and used for subsequent purification and characterization. This procedure produced about 400 units of tumor necrosis factor units/ml of supernatant culture medium.

Human peripheral blood monocytes were also used for the production of tumor necrosis factor. Plateletpheresis residues were obtained from American Red Cross, Boston, MA, and used within 24 h of collection. The initial separation of monocytes from erythrocytes was achieved by centrifugation on Ficoll-Hypaque gradients at 1000g for 30 minutes. Cells collected at the interface were washed three times with phosphate buffered saline. Monocytes derived from each donor were grown separately in 2-liter roller bottles in serum free RPMI 1640 medium at a cell density of 2.5 x $10^6$ cells/ml. 1 $\mu$g/ml each of Staphlococcal enterotoxin B (SEB) and recombinant thymosin $\alpha$-1 were added to the culture and the cells incubated in a humidified atmosphere at 37°C with 10 percent $CO_2$. After 24 - 72h, depending upon the donor, the cell supernatants were harvested and processed in a similar manner to those derived from the HL-60 cell line. Tumor necrosis factor yields from PBL cultures varied widely, depending upon the inducing agents employed. Adding PMA to the induction system described above increased the cytolytic activity of cell supernatants. However, the cell supernatants contained both tumor necrosis factor and lymphotoxin (determination of lymphotoxin or tumor necrosis factor in mixtures of tumor necrosis factor and lymphotoxin was made by conducting the cell lytic assay with test samples preincubated with rabbit neutralizing antibody to tumor necrosis factor or lymphotoxin and determining residual activity in the L-929 cell lytic assay).

Example 3

Controlled Pore Glass Bead Chromatography

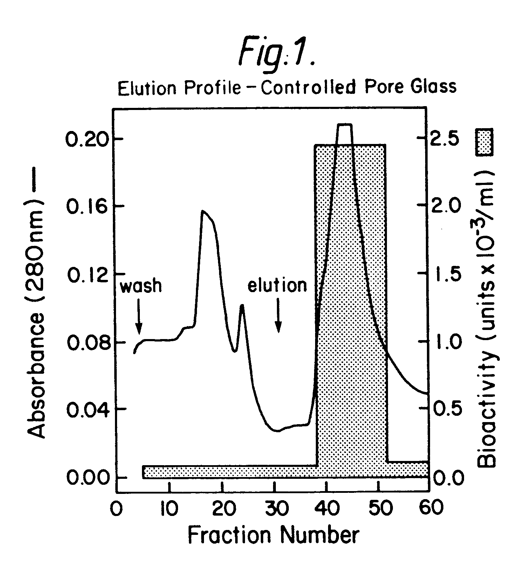

Tumor necrosis factor activity from cell culture was batch absorbed to controlled pore glass beads (Catalogue No. CPG 00350, Electro-Nucleonics, Fairfield, NJ) equilibrated with 10 mM sodium phosphate buffer, pH 8.0, by constant stirring at 4°C. One hundred ml of glass beads were used per 5 liters of medium. After stirring for one hour, beads were allowed to settle and the supernatant was decanted off. The beads were then poured into a 5 x 50 cm column at room temperature and washed with 10 mM sodium phosphate buffer, pH 8.0, containing 1M NaCl. The tumor necrosis factor activity was eluted from glass beads with 20 percent ethylene glycol in 10 mM sodium phosphate buffer, pH 8.0, containing 1M NaCl. The elution profile of the HL-60 supernatant from the column is shown in Fig. 1.

Example 4

DEAE Cellulose Chromatography

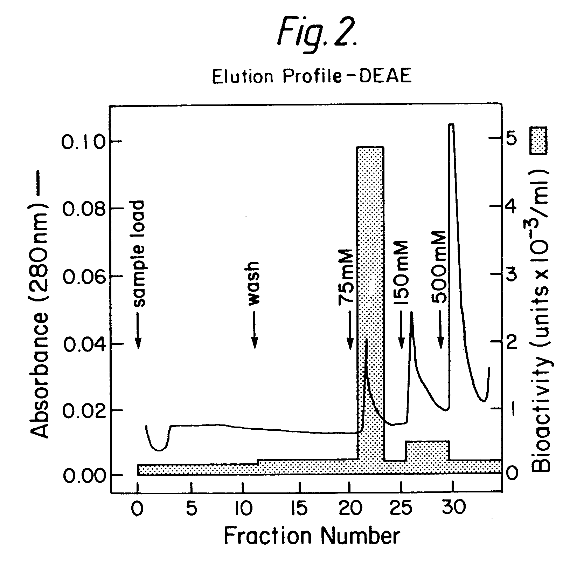

The eluate from Example 3 was directly applied to a DEAE cellulose 53 (Whatman) column (2.5 x 20 cm) equilibrated with 10 mM sodium phosphate buffer at pH 8.0 and 0.01 percent Tween 20, at a flow rate of approximately 500 ml/h. After the flow rate of the column was adjusted to 100 ml/h, 4.2 x $10^6$ units of tumor necrosis factor in 1,080 ml of sample was loaded at 4°C, the column was washed with equilibration buffer and eluted with step-up gradients of 75 mM 150 mM and 500 mM sodium chloride in 10 mM phosphate buffer (pH 8.0). The eluate was monitored for absorbance at 280 nm and tumor necrosis factor activity as a function of elution fractions. The results are shown in Fig. 2.

Example 5

Fast Protein Liquid Chromatography

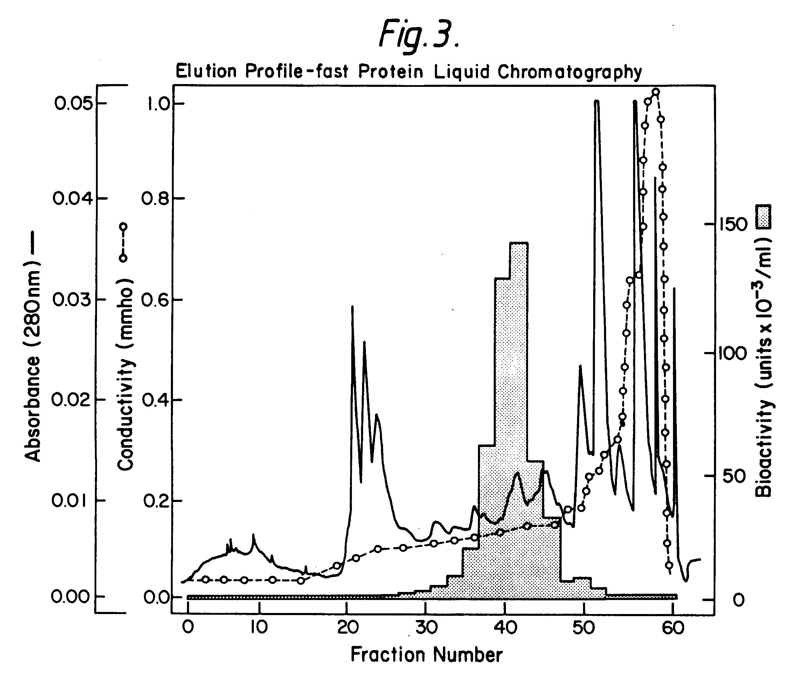

The tumor necrosis factor active fraction from Example 4 was concentrated and dialyzed against 20 mM Tris HCl, pH 8.0, containing 0.01 percent Tween 20 and 1 mM sodium azide (buffer A) in an Amicon stir cell using a YM-10 membrane or other dialysis membrane with a molecular weight cut-off below that of TNF. The membrane was washed twice with buffer A. A quaternary ammonium-group substituted Sepharose bead column (9.8 $\mu$M beads in a 5 x 0.5 cm column; sold as Mono Q resin, Pharmacia) in a fast protein liquid chromatography (FPLC) unit (Pharmacia) equipped with a gradient programmer (GP-250) and two pumps (P-500), was preequilibrated with dialysis buffers via a superloop at a flow rate of 1 ml/min as further described in J. Richey, "American Laboratory" October 1982, page 1. The pooled washes and dialysis concentrate were loaded on the column, the column was washed with buffer A and then eluted with a linear gradient of 40-75 mM sodium chloride in buffer A. Linear gradients were programmed as follows: 0-5 min equilibration buffer; 5.1-15 min, 25 mM NaCl; 15.1-25 min, 40 mM NaCl; 25-60 min, 40-75 mM NaCl linear gradient; 60-65 min, 75 mM NaCl; 65.1-70 min, 100 mM NaCl; 70-80 min, 100-1000 mM NaCl linear gradients; 80-90 min, 100 mM NaCl; 90.1-110 min, equilibration buffer. The effluent was taken as 2 ml per fractions and monitored for absorbance at 280 nm, conductivity, and for tumor necrosis factor activity. The results are shown in Fig. 3.

Example 6

Chromatofocusing

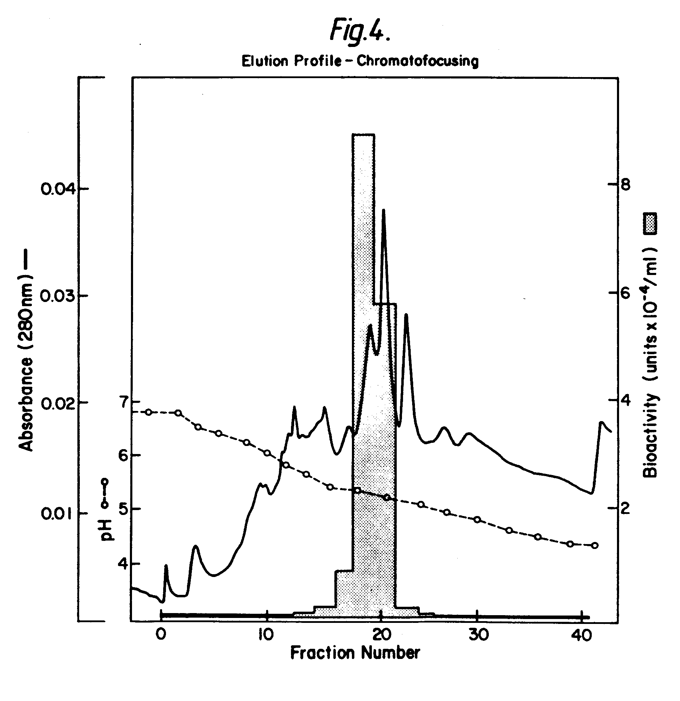

Chromatofocusing was performed using a Pharmacia Mono P column (20 x 0.5 cm) in an FPLC system as in Example 5. The biologically active (tumor necrosis factor) fraction eluted in fraction numbers 37 to 45 from Example 5 was concentrated and dialyzed on an Amicon stir cell with a YM-10 membrane against the column equilibration buffer, i.e., 0.025 M bis-Tris HCl, pH 6.7. The sample was loaded onto the Mono P column at room temperature via a superloop at a flow rate of 1 ml/min. The column was washed with equilibration buffer until the absorbance at 280 nm returned to baseline and then eluted with a linear pH gradient established by washing the column with 7.5 percent polybuffer 74 at pH 4.7 (Pharmacia). One ml fractions were collected and the absorbance at 280 nm and pH of the effluent recorded. The results are shown in Fig. 4. As can be seen from Fig. 4, the isoelectric point of tumor necrosis factor was about 5.3.

Example 7

Preparative SDS-Polyacrylamide Gel Electrophoresis

Fifteen percent polyacrylamide gels (11 x 16 cm) with a thickness of 1.5-3.0 mm were prepared according to a modification of the procedure of U. Laemmli, 1970, "Nature" 227:680-685. Both resolving and stacking gels contained 0.1 percent SDS and 0.05 percent Tween 20. Other buffers and concentration of cross linking reagent were the same as for analytical SDS-PAGE gels. Tumor necrosis factor active fractions from Examples 5 or 6 step were pooled, concentrated and dialyzed against 6.25 mM Tris HCl, pH 7.0, containing 0.005 percent SDS on an Amicon stir cell using a YM-10 membrane. After removal of the dialyzed concentrate, the membrane was washed three times with a small volume of sample buffer (0.2 percent SDS, 0.02 percent Tween 20, 30 percent glycerol, 0.03M Tris HCl, pH 6.8, 0.005 percent tracking dye). The dialyzed concentrate and washes were pooled (total volume 1-4 ml), mercaptoethanol optionally added to establish SDS PAGE reducing conditions, and the sample loaded into a large well cast in the stacking gel. Small wells adjacent to the sample well were used for prestained molecular weight markers phosphorylase-a (94K), bovine serum albumin (67K), ovalbumin (43K), carbonic anhydrase (30K), soybean trypsin inhibitor (20K) and Lysozyme (14.4K). The gels were run in a Biorad vertical electrophoresis system cooled to 12°C, at a constant current of 20 mA per mm of gel thickness, until the tracking dye reached the bottom of the gel.

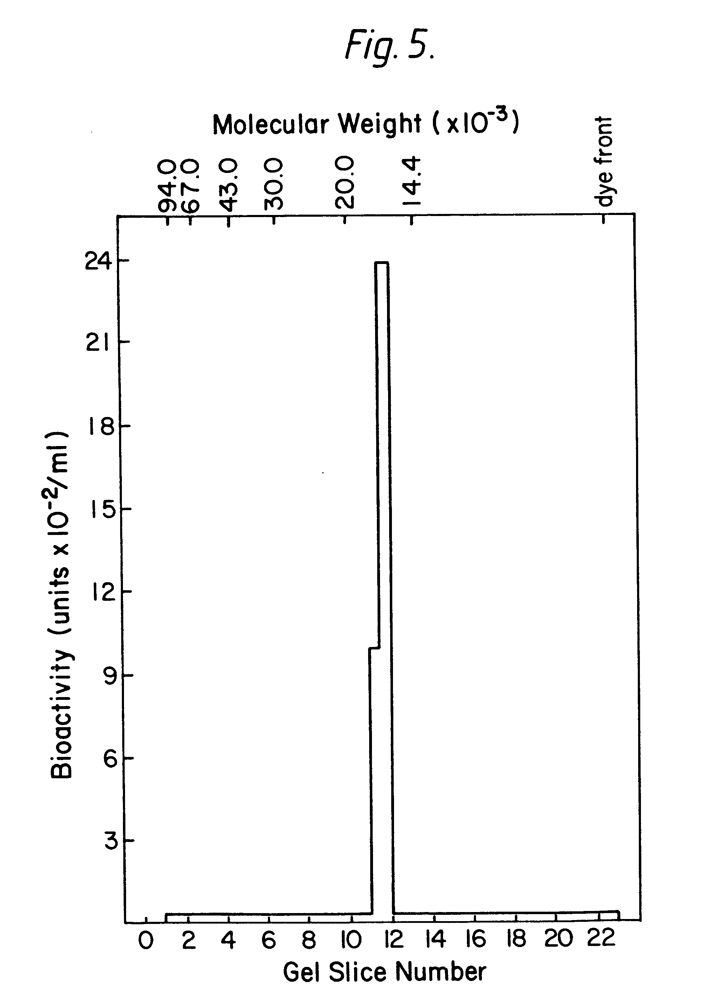

Following electrophoresis, one of the glass plates was removed from the gel, and the positions of the molecular weight markers were noted. The lane containing the applied tumor necrosis factor sample was then cut into 0.25 cm sections in accord with the molecular weights of the marker proteins. These gel slices were then placed in polypropylene tubes containing 1-2 ml of 10 mM ammonium bicarbonate and 0.01 percent Tween 20, pH 8, and allowed to elute for 16 h at 4°C. The eluates were then assayed for tumor necrosis factor activity and the results shown in Fig. 5. The tumor necrosis factor molecular weight on SDS gel was about 17,000, whether under reducing or nonreducing conditions, thus indicating a single chain molecule.

The protein was recovered from the eluate of gel slices free of salts and low molecular weight substances by the following treatment: Small columns were prepared containing 0.2 ml Sep-pak C18 resin, which had been pre-washed with acetonitrile, 1-propanol, 1 percent trifluoroacetic acid (TFA), and distilled water and then equilibrated with 10 mM ammonium bicarbonate containing 0.01 percent Tween-20, pH 8.0. The gel eluate was loaded onto the column and the effluent collected. The resin was then washed with approximately 5 ml each of distilled water and 0.1 percent TFA, to remove free amino acids and buffer salts. Tumor necrosis factor was eluted from the resin with 1 ml of 50 percent 1-propanol in 0.1 percent TFA. Further elutions with 1 ml each of 50 percent 1-propanol in 1 percent TFA, and 99 percent 1-propanol in 1 percent TFA were also performed, but the protein was usually eluted with the first buffer. Approximately 80 percent of the tumor necrosis factor bioactivity was inactivated in this step. While the tumor necrosis factor so obtained can be employed for sequence analysis it is preferred that the HPLC effluent described below in Example 8 be used for this purpose.

Example 8

High Pressure Liquid Chromatography

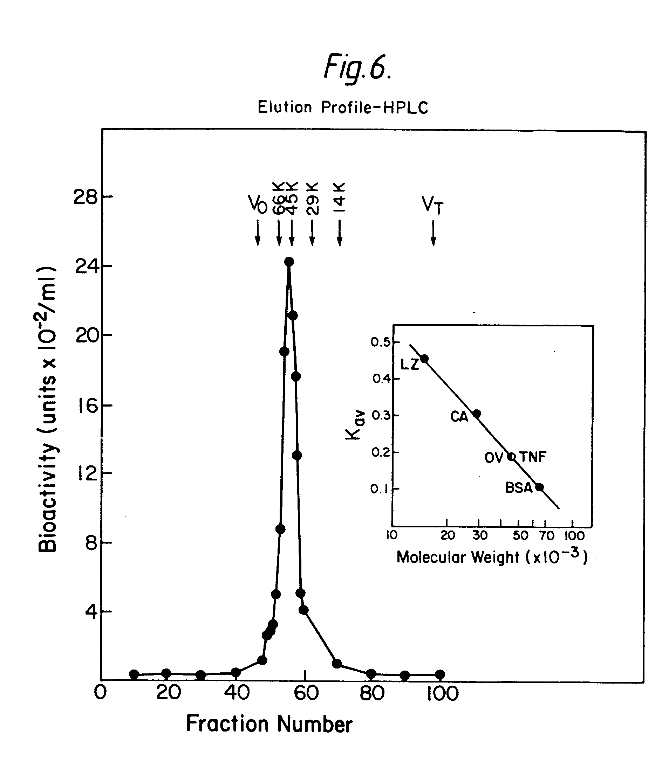

The molecular weight of native intact tumor necrosis factor was determined by high pressure gel permeation chromatography. The latter was carried out at room temperature using a TSK G2000 SW gel HPLC column (Alltech Associates, Deerfield, IL)(7.5 x 60 mm). A one ml sample of purified tumor necrosis factor from Example 5 containing approximately 1μg of protein and 15,600 units of activity was isocratically eluted from the gel column at a flow rate of 0.5 ml/min, with 0.2M sodium phosphate buffer, pH 7.0. The column was calibrated with bovine serum albumin (MW 66,000), ovalbumin (MW 45,000), bovine carbonic anhydrase B (MW 29,000), and lysozyme (MW 14,300). One ml fractions were obtained and assayed for tumor necrosis factor activity. The fractions showing tumor necrosis factor activity eluted consistent with a molecular weight of 45,000 ± 6,000 (Fig. 6).

Example 9

Reverse-phase HPLC

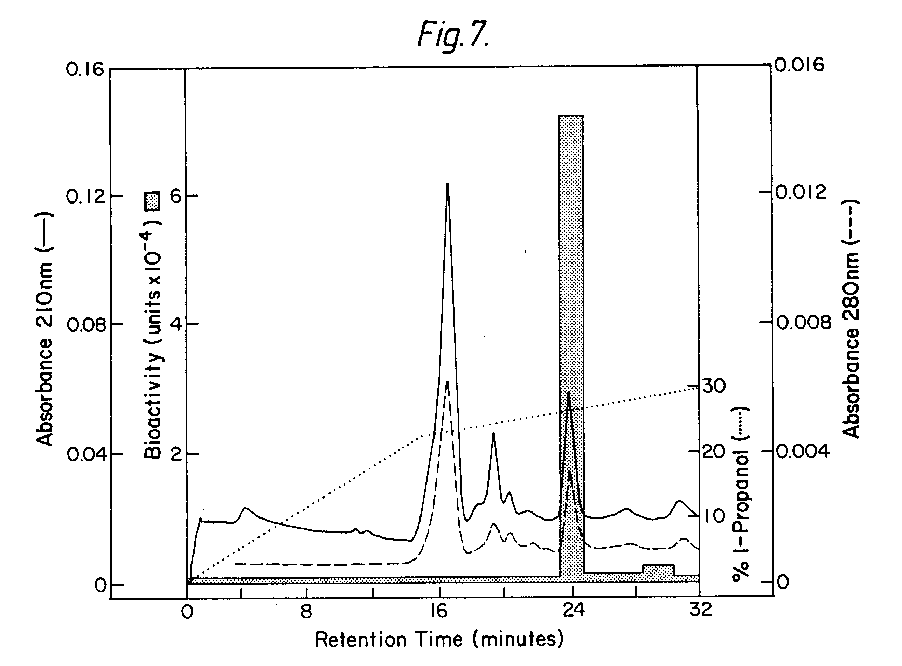

Tumor necrosis factor also was purified by reverse-phase HPLC using C4 Synchropak columns on Water's Associates, Inc. chromatograph system as described previously (W. Kohr et al, 1982, Anal. Biochem, 122: 348-359). Protein peaks were detected at 210 nm and at 280 nm after elution with a linear gradient of 1 to 23 percent v/v 1-propanol in 0.1 percent aqueous TFA in the first 15 minutes and 23 - 30 percent v/v 1-propanol in 0.1 percent TFA for the next 15 minutes at a flow rate of one ml per minute. The peaks were assayed for cytolytic activity. The organic solvents employed in eluting tumor necrosis factor from the C4 column reduced tumor necrosis factor activity about 80 percent. Tumor necrosis factor purified by this method was dried under vacuum, and then processed for amino acid analysis and sequencing. The results are recorded in Fig. 7. Fig. 7 shows that the tumor necrosis factor obtained in the effluent from Example 5 contained biologically inactive protein contaminants eluting at about 16 and 19 minutes retention time. The bioactive effluent from C4-RP-HPLC was substantially homogeneous, by the criteria of amino terminal sequence.

Example 10

Determination of Partial Amino Acid Sequence for Tumor Necrosis Factor

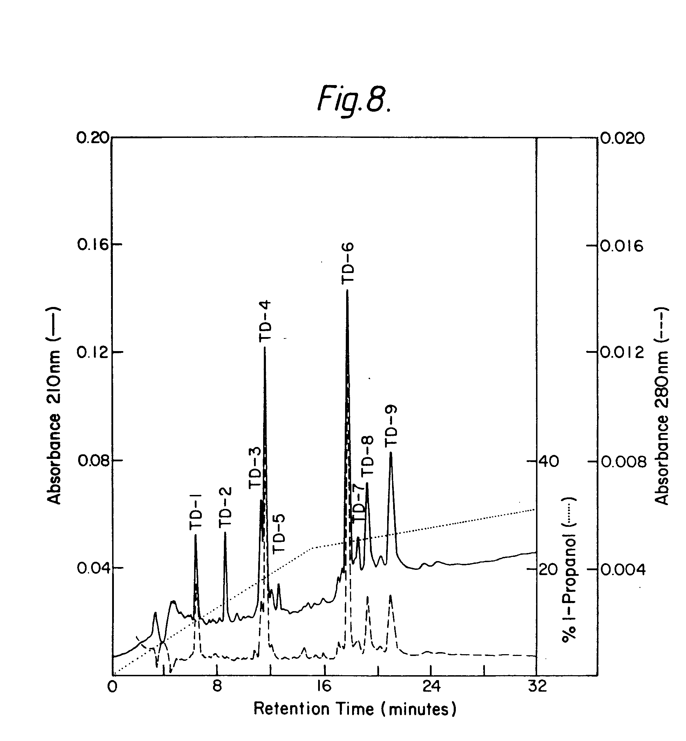

Tumor necrosis factor was trypsin digested as follows: Homogeneous tumor necrosis factor from Example 9 was dissolved, dried down and redisolved in 100 mM of ammonium bicarbonate buffer pH 8.0 containing 5 percent w/w TPCK trypsin (Worthington Biochemicals), 1mM CaCl₂ and 0.01 percent Tween-20 at an enzyme to substrate ratio of 1:20, incubated for 6 hours at 37°C, an additional 5 percent by w/w of trypsin added and the hydrolysis mixture further incubated for 12 hours at 37°C. The reaction mixture was applied onto C4 HPLC as described above in order to separate the peptide fragments. The results are shown in Fig. 8. A total of 9 fragments were observed (fragments 2 and 2' eluted together at the peak designated T2 in Fig. 8). An additional tenth fragment is believed to not be retained by the column. Amino acid sequences for intact tumor necrosis factor from Examples 8 and 9 and the trypsin hydrolysis fragments obtained in this Example were determined by automated sequential Edman degradation using a modified Beckman sequencer model 890B equipped with cold traps. Polybrene (1.25 mg) was used as a carrier in the cup. Based on the amino acid composition of the intact molecule, the molecular weight of intact tumor necrosis factor is 17,100. This figure was consistent with the SDS-PAGE data and constitutes confirmation of the absence of glycosylation.

Example 11

Synergistic Action of Tumor Necrosis Factor and Gamma Interferon

Cells of the murine melanoma B16, (Mason Research, Worcester, MA.) a cell line of C57B1/6 origin, were seeded in a microtiter plate at 5,000 cells/well and incubated for 4 hours at 37°C in a 5 percent $CO_2$-humidified incubator before the addition of the lymphokines. Tumor necrosis factor obtained from Example 1 was purified to substantial homogeneity by HPLC and quantitated by its activity in the above-described bioassay for cytolysis of L929 cells. Similarly, purified recombinant murine gamma interferon (P. Gray et al., 1983, "Proc. Natl. Acad. Sci. U.S.A." 80: 5842-5846) was assayed by its antiviral activity against EMC-infected L cells (D. Goeddel et al., 1980, "Nature" (London) 287: 411-416). Murine gamma interferon and human tumor necrosis factor were separately diluted to the dilutions shown in Fig. 10. Gamma interferon was added first to the designated wells, and diluted tumor necrosis factor was added immediately thereafter, to a final volume of 0.2 ml/well. At the end of 72 hours of incubation, the cells were stained with 0.5 percent crystal violet in 20 percent methanol. The results are shown in Fig. 10. B16 is relatively resistant to either tumor necrosis factor or IFN-$\gamma$ alone; at 1,000 units/ml of tumor necrosis factor no visually detectable cytolysis was observed. However, addition of very small amounts of gamma interferon (as little as 5 units/ml) resulted in cytolysis.

Example 12

Messenger RNA Isolation

Total RNA from HL-60 cell cultures (4 hours after PMA induction) or peripheral blood monocytes cultured as described in Example 2 was extracted essentially as reported by Ward et al., 1972, "J. Virol." 9: 61. Cells were pelleted by centrifugation and then resuspended in 10 mM NaCl, 10 mM Tris-HCl pH 7.5, 1.5 mM $MgCl_2$. Cells were lysed by the addition of NP-40 (1 percent final concentration), and nuclei were pelleted by centrifugation. The supernatant contained the total RNA which was further purified by multiple phenol and chloroform extractions. The aqueous phase was made 0.2 M in NaCl and then total RNA was precipitated by the addition of two volumes of ethanol. A typical yield from 1 gram of cultured cells was about 6 milligrams of total RNA. Polyadenylated mRNA (about 100 $\mu$g) was obtained on oligo (dT) cellulose by the method of H. Aviv et al., 1972, "Proc. Natl. Acad. Sci. U.S.A." 69: 1408-1412.

Example 13

cDNA Library

7.5 $\mu$g of poly(A)$^+$ mRNA from Example 12 was converted to double stranded cDNA by the successive action of reverse transcriptase, DNA polymerase Klenow fragment, and S1 nuclease (P. Gray et al., 1982, "Nature" 295: 503-508; M. Wickers et al., 1978, "J. Biol. Chem." 253: 2483-2495). About 80 ng of cDNA having greater than 600 bp in length were isolated from a polyacrylamide gel.

The synthetic DNA sequence

```
5'AATTCATGCGTTCTTACAG 3'
3'GTACGCAAGAATGTC 5'
```

was ligated to the cDNA to create EcoRI cohesive termini. As is conventional in the art, the adaptor was synthesized chemically as two separate strands, the 5' end of one of the strands was phosphorylated with polynucleotide kinase and the two strands annealed. The cDNA (20 ng) was then re-isolated from a polyacrylamide gel, inserted by ligation into EcoRI-digested λgt-10, packaged into phage particles and propagated in E. coli strain C600 hfl (Huynh et al., 1984, Practical Approaches in Biochemistry, IRL Press Ltd., Oxford England) or other known strain suitable for lamda phage propagation. A cDNA library of about 200,000 independent clones was obtained.

Example 14

Preparation of a Deoxyoligonucleotide Probe for Tumor Necrosis Factor cDNA

A 42 nucleotide DNA hybridization probe, based upon the preliminary amino acid sequence of tumor necrosis factor tryptic peptide TD-6 (E-T-P-E-G-A-E-A-K-P-W-Y-E-K-) was designed on the basis of published codon usage frequencies (R. Grantham et al., 1981 "Nucleic Acids Res." 9: 43-74), and the codon bias of human IFN-γ (P. Gray et al., 1982, "Nature" 295: 503-508), and human lymphotoxin The preliminary sequence was in error (the final K should have been P). Nonetheless, this sequence led to a successful probe. The synthetic probe had the sequence
5' dGAAACCCCTGAAGGGGCTGAAGCCAAGCCCTGGTATGAAAAG 3' and was synthesized by the method of R. Crea et al., 1980, "Nucleic Acids Res." 8: 2331-2348. The probe was phosphorylated with (γ-$^{32}$P) ATP and T4 polynucleotide kinase as described previously (Goeddel et al., 1979, "Nature" 281: 544).

Example 15

Identification of a cDNA Clone Containing Tumor Necrosis Factor Coding Sequences

About 200,000 recombinant phage from the λgt 10 cDNA library were screened by DNA hybridization using the $^{32}$P-labelled 42-mer from Example 14 under conditions of low stringency in accordance with A. Ullrich et al., 1984, "EMBO J." 3: 361-364 (or alternatively P. Gray et al., 1983, "Proc. Natl. Acad. Sci. U.S.A." 80: 5842-5846, S. Anderson et al., 1983, "Proc. Natl. Acad. Sci. U.S.A." 80: 6836-6842 and M. Jaye et al., 1983, "Nucleic Acids Res." 11: 2325-2335). Nine distinct clones hybridized with the probe and were plaque purified. Then $^{32}$P-labelled cDNA was prepared using mRNA from uninduced HL-60 cells. DNA from seven of these nine phage clones failed to hybridize with this "uninduced" probe and were therefore judged to be candidates for tumor necrosis factor cDNA sequences. The cDNA clone containing the largest insert was designated λ42-4. This insert was sequenced by the dideoxy chain termination method (A. Smith, 1980, "Methods in Enzymology" 65: 560-580 and F. Sanger et al., 1977, "Proc. Natl. Acad. Sci. U.S.A." 74: 5463-5467) after subcloning into the vector M13mp8 (J. Messing et al., 1981, "Nucleic Acids Res." 9: 309-321).

The cDNA sequence obtained for λ42-4 contained the entire coding region for mature tumor necrosis factor plus a portion of its signal peptide. The correct orientation and reading frame of the DNA was deduced by comparison with the amino acid sequence of tryptic peptide T4 of tumor necrosis factor. The amino terminal valine residue of tumor necrosis factor determined by protein sequencing is indicated as amino acid 1, and is followed by 156 additional amino acids before a stop codon in reading phase is encountered. The calculated molecular weight is 17,356 daltons.

Example 16

Identification of a cDNA Clone Containing Complete PreTNF Coding Sequences

The cDNA clone λ42-4 contains the entire coding region of mature TNF but lacks a complete signal peptide coding sequence as evidenced by its lack of an initiation codon. To obtain the missing sequence information, the hexadecanucleotide primer dTGGATGTTCGTCCTCC (complimentary to nucleotides 855 to 870, Fig. 10) was chemically synthesized. This primer was annealed to mRNA from Example 12 and then cDNA was synthesized using the method of Example 13. A new library of about 200,000 cDNA clones was prepared in λgt10 following the method described in Example 13. This library was screened by hybridization analysis using as a probe the λ42-4 cDNA insert which had been $^{32}$P-labelled. Sixteen positive clones were obtained, the longest (λ16-4) of which contained a cDNA insert extending 337 bp further 5' than the λ42-4 insert. The composite sequence of the TNF cDNA inserts of λ16-4 (nucleotides 1-870) and λ42-4 (nucleotides 337-1643) is shown in Figure 10.

Example 17

Construction of an Expression Vector for Direct Expression of Tumor Necrosis Factor

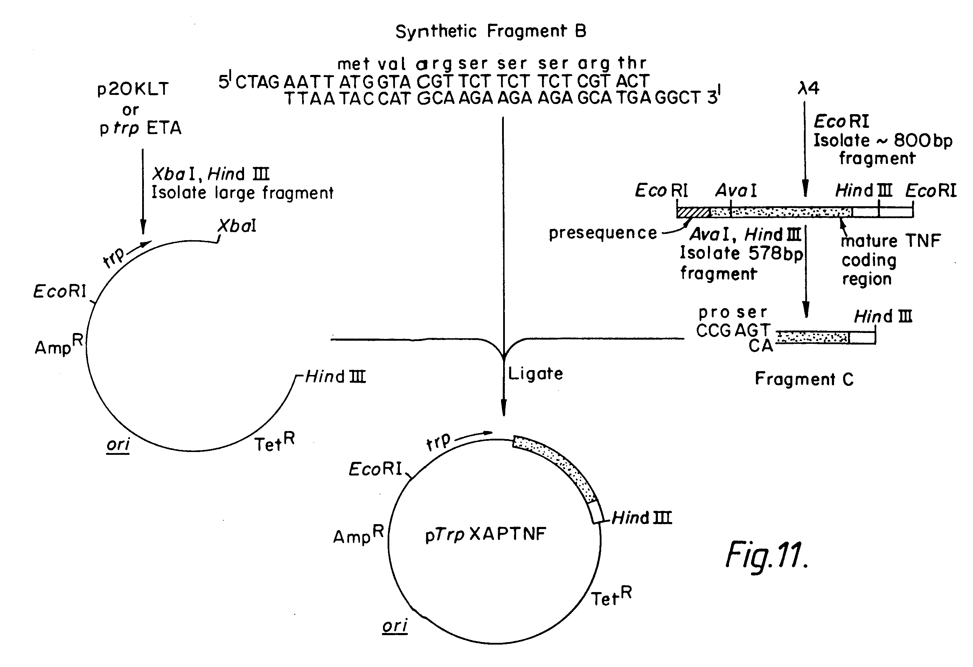

The procedure used to express the cDNA sequence for tumor necrosis factor obtained in Example 15 is set forth in Fig. 11. Phage λ42-4 from Example 15, containing the entire mature tumor necrosis factor coding sequence and a portion of the putative tumor necrosis factor secretory leader, was digested with EcoRI and an approximately 800 bp fragment containing the tumor necrosis factor coding region was recovered. This fragment was digested with Ava I and Hind III and a 578 bp fragment (designated "C" in Fig. 11) recovered. This fragment encodes tumor necrosis factor amino acids 8-157.

Two synthetic deoxyoligonucleotides (designated fragment "B" in Fig. 11) were prepared (see the construction of the adaptor sequence in Example 13) which incorporated an Xba I cohesive terminus at the 5' end, an Ava I cohesive terminus at the 3' end, a met initiation codon and codons for the first seven amino terminal amino acids of tumor necrosis factor. The codons for these amino acids were chosen on the basis of E. coli preference. The AATT sequence upstream from the start codon was selected to properly space the start codon from the trp ribosome binding sequence and, in combination with the amino acid codons, to eliminate a potential messenger RNA loop.

Segments B and C are then joined in a three-fold ligation with a pBR322 derivative containing the trp promoter sequence with the Shine-Dalgarno sequence of the trp leader peptide (European Patent Application Publn. No. 36776). The derivative is obtained or designed to contain unique Xba I and Hind III sites between the trp promoter and the Tet$^R$ gene. Either pLTtrp1 (Gray et al., 1984, "Nature" 312: 721-724) or ptrpETA (Gray et al., 1984, "Proc. Natl. Acad. Sci. U.S.A." 81: 2645-2649) are suitable starting vectors of this type, although others may be constructed from pBR322, the trp promoter and any required synthetic linkers. Both pBR322 and plasmids containing the trp promoter are publicly available. The pBR322 portion of the vector chosen may have the AvaI-PvuII segment from bp 1424 to 2065 deleted (designated "XAP" in the plasmid name). Any of the foregoing plasmids are simultaneously digested with Xba I and Hind III and the large vector fragment recovered. This fragment, and fragments B and C are ligated with T4 DNA ligase and the ligation mixture used to transform E. coli 294 (ATCC 31446). Ampicillin resistant colonies were selected, plasmid DNA recovered and characterized by restriction endonuclease mapping and DNA sequencing. pTrpXAPTNF was obtained which contained inserts B and C.

Example 18

Tumor Necrosis Factor Expression in E. coli

E. coli ATCC 31446 which had been transformed with pTNFtrp was grown in M9 medium containing 20 μg/ml ampicillin and the culture grown to an $A_{550} = 0.3$. Indole acetic acid was added to give a final concentration of 20 μg/ml and the culture grown to $A_{550} = 1$. 10 ml of cells were concentrated and resuspended in phosphate buffered saline. The cells were sonicated and diluted for tumor necrosis factor determination in the Example 1 assay. Approximately $10^5$ units of activity were obtained per ml of culture. This activity was neutralized by preincubation with rabbit antisera from rabbits immunized against human tumor necrosis factor.

Example 19

Tumor Necrosis Factor Expression in E. coli

This method is preferred over that of Example 18.

Preferably, the host for use with the above vectors is a non-revertable tonA E. coli strain. Such strains are bacteriophage resistant and therefore far more suitable for large scale culture than wild-type strains. Following is a description of a suitable method for generating such a strain. E. coli W3110 is transduced with λ::Tn10, a lambda bacteriophage containing the transposable element Tn10, to generate a Tn10 hop pool of E. coli W3110. (N. Klecker et al., 1977 "J. Mol. Biol." 116: 125).

The E. coli W3110::Tn10 hop pool is grown in L broth at 37°C to a cell density of about 1 x 10$^9$/ml. 0.5 ml of the culture is centrifuged and the pellet is resuspended in 0.2 mls of a λphi80 (or T1) lysate containing 7.0 x 10$^9$ pfu. The phage is allowed to adsorb for 30 minutes at 37°C. The suspension is then spread on EMB plates supplemented with tetracycline (15 μg/ml). After an overnight incubation at 37°C, the light pink colonies are pooled in 3 ml of L broth, grown overnight at 37°C, washed twice, and resuspended in L broth. This culture is infected with bacteriophage P1 kc, and the phage lysate recovered (J. Miller, 1972, Experiments in Molecular Biology, Cold Spring Harbor Laboratory, p 304).

E. coli AT982 (no. 4546, E. coli Genetic Stock Center, New Haven, Conn.) is transduced to tetracycline resistance by this P1 kc lysate. Transductants are selected on L broth plates supplemented with tetracycline (15 $\mu$g/ml) and (40 $\mu$g/ml) dap (diaminopimelic acid). The resulting transductants are screened for tetracycline resistance and the regeneration of the dap gene (dap$^+$, tet$^R$). dap$^+$, tet$^R$ transductants are then tested for $\lambda$phi80 (or T1) resistance.

P1 kc lysates are then made on several dap$^+$, tet$^R$,$\lambda$phi80 (or T1) resistant strains. The lysates are used to transduce E. coli W3110 to tetracycline resistance. The transductants are screened and selected for $\lambda$phi80 (or T1) resistance.

Tetracycline sensitive isolates are selected from the W3110 fhuA::Tn10-$\lambda$phi80R transductants (S. Naloy et al., 1981 "J. Bact." 145: 1110). These isolates are checked for phage $\lambda$phi80 resistance and tetracycline sensitivity after single colony purification.

DNA is isolated from several tetracycline sensitive $\lambda$phi80 phage resistant mutants and digested with SstII. The SstII digested DNA is characterized by the Southern blot procedure using radioactively labelled and SstII digested $\lambda$::Tn10 DNA as a probe to determine if the Tn10 has excised (R. Davis et al., 1980, Advanced Bacterial Genetics, Cold Spring Harbor Laboratory). One of the tetracycline sensitive isolates is shown to have lost two of the Tn10 hybridization bands as compared to the hybridization between DNA from the $\lambda$::Tn10 and the parental W3110 fhuA::Tn10$\lambda$phi80R. A third hybridization band has an altered mobility indicating that a deletion caused by the imprecise excision of Tn10 has occurred.

SDS-gel electrophoresis of outer membrane preparations from the strain with an imprecise Tn10 excision reveal that the band assumed to be the fhuA protein has an altered electrophoretic mobility as compared to the wild-type fhuA protein. The resulting protein is non-functional as a $\lambda$phi80 phage receptor protein. A second independent strain which also has undergone imprecise excision of Tn10 shows no fhuA protein on the SDA gel.

Neither of these strains demonstrate reversion to tetracycline resistance or to $\lambda$phi80 susceptability indicating that there is an imprecise excision of all or part of the Tn10 transposon together with either a partial or complete deletion of the fhuA gene. Preferably, one of such W3110 strains (NL106) is used as a host for the TNF-encoding vehicles described elsewhere herein.

NL106 was transformed with ptrpXAPTNF and inoculated into 10 liters of a pH 7.4 medium having the following formula:

| Component | gms/L |
|---|---|
| $(NH_4)_2SO_4$ | 5.0 |
| $K_2HPO_4$ | 6.0 |
| $NaH_2PO_4$ | 3.0 |
| Na Citrate | 1.0 |
| L-Tryptophan | 0.2 |
| NZ Amine AS | 4.0 |
| Yeast Extract | 4.0 |
| $MgSO_4$ | 1.2 |
| Glucose | 25.0 |
| Trace Element solution (Fe, Zn, Co, Mo, Cu, B and Mn ions) | 0.5 ml |
| Tetracycline | 1.0 mg |

Glucose was fed to the culture at a rate of 1 gm/minute when the $A_{550}$ of the culture reached about 20. The fermentation was conducted at 37°C until an $A_{550}$ of 136 was reached (about 20 hours). The culture is centrifuged to form a cell paste and the paste then extracted for 30 min. at pH 8.0 and room temperature with a buffer containing 50 mM tris, 10 mM EDTA, 1000 mM NaCl, 2000 mM urea and 0.1 percent beta-mercaptoethanol. The extract was diluted and assayed as described in Example 1. $1\times10^8$ units of tumor necrosis factor activity in this assay was established as equivalent to 1 mg of tumor necrosis factor. Up to about 2 grams of tumor necrosis factor were obtained per liter of culture. Amino terminal sequencing demonstrated that about from 75 to 86 percent by weight was valyl amino-terminal (mature) tumor necrosis factor, the remainder being met-TNF. Furthermore, in addition to high expression levels the protein was not present in refractile bodies, nor did it otherwise appear to be toxic to the cells as evidenced by the extremely high cell densities that were obtained.

Example 20

Construction and Expression of a Mutant Tumor Necrosis Factor Gene

In this contemplated example, Examples 17-18 were repeated except that the oligonucleotide fragment B was synthesized with a histidine codon CAT in place of the arginine 6 codon CGT. Mutant tumor necrosis was expressed.

Example 21

Construction and Expression of Another Mutant Tumor Necrosis Factor Gene

In this example, the procedure of Examples 17-18 was repeated with an oligonucleotide fragment B encoding leucine (CTT) in place of the residue 2 arginine codon. About 1200 mg of mature TNF activity were obtained per liter of culture in initial trials. Unprocessed TNF was not detectable in the culture.

Example 22

Construction of a Vector Encoding a Tumor Necrosis Factor Fusion with a Secretory Signal Sequence

The sequence of the E. coli heat stable enterotoxin gene STII is depicted in Picken et al (below). In this Example a fragment containing the STII secretory signal and Shine-Dalgarno sequence was ligated downstream of the E. coli alkaline phosphatase promoter. The STII signal is followed in the 3' direction with a synthetic oligonucleotide which supplies codons for the initial seven amino-terminal tumor necrosis factor amino acids and the remainder of the coding sequence for tumor necrosis factor. All of the foregoing were assembled in a pBR322 vector.

pWM501 (Picken et al., 1983, "Infection and Immunity" 42(1): 269-275) contains the STII gene depicted. pWM501 was digested with XbaI and NsiI and the approximately 90 bp fragment isolated. This fragment also could be synthesized organically by methods known per se (fragment A).

A pBR322-Trp plasmid as described in Example 17 (p20kLT) was digested with XbaI and HindIII, and the large vector fragment recovered (fragment B). This fragment contains an E. coli origin of replication and a gene conferring the phenotype of ampicillin resistance.

A synthetic oligonucleotide was synthesized as two strands and annealed to yield the following structure (the restriction site sticky ends and amino acids coded for by the oligonucleotide are also indicated).

```
                VAL ARG SER SER SER ARG THR
        5'      GTA CGT TCT TCT TCT CGT ACT        3'

        ACGT CAT ACG AGA AGA AGA GCA TGA GGCT
        NsiI                                AvaI
```

This is designated fragment C.

pTNFtrp from Example 18 was digested with AvaI and HindIII. The 578 bp AvaI-HindIII fragment (fragment D) was recovered. It contains all of the TNF coding sequence except for the first seven amino acids.

A DNA sequence comprising an E. coli alkaline phosphatase (AP) promoter linked to a heterologous Shine-Dalgarno (S.D.) sequence (trp) and having EcoRI and XbaI termini was constructed as follows. A DNA fragment containing a portion of the AP promoter was isolated from the plasmid pHI-1 (H. Inouye et al., 1981, "J. Bacteriol." 146: 668-675), although any appropriate sources containing AP promoter DNA also could be used. pHI-1 was digested with HpaI to open the plasmid, a synthetic EcoRI linker

```
GAATTCGAATTC
CTTAAGCTTAAG
```

ligated to the plasmid and the linkered plasmid digested with an excess of EcoRI to cleave all EcoRI site and with a deficiency of RsaI activity in order to only partially cleave all of the RsaI sites (the EcoRI and RsaI steps also could be done sequentially rather than simultaneously). A 420 bp fragment containing the AP promoter was recovered from the EcoRI-RsaI partial digest.

A trp S.D. sequence was obtained as follows. A plasmid or organism containing the trp promoter (pIFN-beta 2, D. Leung et al., 1984, "Biotechnology" 2: 458-464) was digested with XbaI and RsaI and the 30 bp fragment recovered which contains the trp S.D. sequence. This fragment was ligated to the 420 bp AP promoter fragment to yield a 450 bp EcoRI-XbaI fragment E. Fragment E has the nucleotide sequence

```
EcoRI
GAATTCAACTTCTCCATACTTTGGATAAGGAAATACAGACATGAAAAATCTCATTGCTGAGTTGTTATTT
AAGCTTGCCCAAAAAGAAGAAGAGTCGAAAGAACTGTGTGCGCAGGTAGAAGCTTTGGAGATTATCGTCA
CTGCAATGCTTCGCAATATGGCGCAAAATGACCAACAGCGGTTGATTGATCAGGTAGAGGGGGCGCTGTA
CGAGGTAAAGCCCGATGCCAGCATTCCTGACGACGATACGGAGCTGCTGCGCGATTACGTAAAGAAGTTA
TTGAAGCATCCTCGTCAGTAAAAAGTTAATCTTTTCAACAGCTGTCATAAAGTTGTCACGGCCGAGACTT
                                                    trpS.D. XbaI
ATAGTCGCTTTGTTTTTATTTTTTAATGTATTTGTACGCAAGTTCACGTAAAAAGGGTATCTAGA
```

Fragments A, B, C and D were ligated in a four-part ligation and the ligation mixture used to transform E. coli 294. Transformants were identified by growth on LB plates containing ampicillin. Plasmid trpSTIITNF was isolated from a transformant colony. This plasmid was digested with XbaI and EcoRI to remove the trp promoter, then ligated to the 450 bp long EcoRI-XbaI fragment E containing the E. coli alkaline phosphatase promoter. The resulting plasmid is called pAPSTIITNF.

Example 23

Expression and Processing of a Tumor Necrosis Factor Fusion with a Secretory Signal Sequence

E. coli NL106 was transfected with pAPSTIITNF and innoculated into 10 liters of a pH 7.0 medium having the following formula:

| Component | gms/L |
|---|---|
| $(NH_4)_2SO_4$ | 5.0 |
| $K_2HPO_4$ | 2.6 |
| $NaH_2PO_4$ | 1.3 |
| Na Citrate | 1.0 |
| KCl | 1.5 |
| NZ Amine AS | 5.0 |
| Yeast Extract | 2.0 |
| $MgSO_4$ | 1.2 |
| Glucose | 25.0 |
| Trace Element solution (Fe, Zn, Co, Mo, Cu, B and Mn ions) | 0.5 ml |
| Ampicillin | 20.0 mg |

The culture was conducted in the same fashion as described above in Example 19, except that an $A_{550}$ of 140 was reached. The culture at this point contained about 400 mg of tumor necrosis factor/liter, about 70-80 percent by weight of which was properly processed to the mature protein as estimated from electrophoresis gels. Approximately the same activity of tumor necrosis factor was recovered upon whole cell extraction by the method used in Example 19 as was recovered by osmotic shock of the cells.

Example 24

Construction and Expression of Additional Tumor Necrosis Factor Derivatives

Mutant derivatives of the Fig. 10 TNF amino acid sequence are prepared to satisfy one or more of at least the following objectives: To increase in vivo half life, increase cytolytic activity, increase the net differential cytolytic activity on tumor cells versus normal cells, prepare TNF immunogens for making anti-TNF antibodies for diagnostic uses, to engineer unique sites for covalent modification (for example where enzyme labels are covalently bound in the preparation of EMIT or ELISA diagnostic reagents), and to change the physical properties of the TNF, e.g. its solubility, pI and the like. The determination of the desired activity in selected derivatives is determined by routine screening in suitable assays known per se.

Preferably, TNF and its derivatives will not include TNF having a sequence corresponding to that of nonprimates, also, it preferably will not have the amino terminii of nonprimates, e.g. the desValArg amino terminus characteristic of what has been referred to as rabbit tumor necrosis factor, nor the amino terminus of the TNF gene identified to have the sequence Val-Arg-Ser-Arg-Thr-Pro-Ser-Asp-Lys-Pro-Val-Ala-Val-Ser-Val-Ala-Asn-Pro-Aln-Ala-Glu-Gly- (Wang et al., "Science" 228: 149-154, 1985).

The following method, based on J. Adelman et al., "DNA" 2(3): 183-193, is generally applicable to the construction and expression of any mutant TNF DNA sequences containing silent mutations or encoding mutant TNF. In order to avoid redundancy, representative derivatives are illustrated in which Arg 32 is converted to histidyl (a substitution), His 73 is deleted (a deletion mutation) and leucyl is fused to Leu 157 (an insertion). It should be understood, however, that all other mutant species are generated in the same fashion.

Other methods suitable for creating silent or expressed mutations in the tumor necrosis factor-encoding DNA are known to those in the art. For example, mutant DNA is constructed by simply chemically synthesizing the entire sequence or by synthesizing a portion of the sequence and ligating the fragment into the remainder of the required DNA. Chemical DNA synthesis is advantageous when the artisan wishes to prepare the mutant directly without first obtaining from natural sources the DNA encoding tumor necrosis factor. Ordinarily, however, the starting DNA will encode the natural amino acid sequence, including its allelic variants, and it will be desired to prepare therefrom certain derivative mutants.

It is desirable in preparing DNA encoding mutant TNF derivatives that codon changes not be made which create the opportunity for mRNA transcribed therefrom to form strong stem-and-loop structures. Avoidance of such structures will generally result in higher yields. In addition, transformant host-preferred codons should be employed for the same reason.

Suitable starting DNA is the EcoRI-HindIII fragment of pTrpXAPTNF (Example 17) obtained by sequential digestion with EcoRI and HindIII, followed by isolation of the TNF gene-containing fragment. This fragment includes the trp promoter and structural gene for methionyl-tumor necrosis factor. To obtain a single-stranded copy of this gene suitable for mutagenesis, the EcoRI-HindIII fragment is cloned into the polylinker site of phage M13 mp8 RF-DNA (J. Messing et al., 1982, "Gene" 19: 269-276; "RF" means the replicative form of the phage; this phage is commercially available). An aliquot of the EcoRI-HindIII digestion mixture is added to a ligation reaction containing 10 ng of M13 mp8 RF-DNA which had been digested previously with EcoRI and Hind III. After incubation at room temperature for 2 hr, the ligation mixture is used to transform E. coli JM103 (a commercially available strain; JM101 can also be used). Transformed cells are plated with top agar containing X-GAL (dibromo-dichloro-indolyl-galactoside) and IPTG (isopropylthiogalactoside). Bacterial cultures (1 ml) infected with phage picked from colorless plaques are used to isolate M13 mp8/TNF RF-DNA by a miniscreen procedure (Birnboim and Doly, 1980, "Nuc. Acids Res." 7: 1513-1523). The resulting recombinant phage M13 mp8/TNF carries the coding strand for the TNF gene.

For site-directed mutagenesis, oligodeoxyribonucleotides (mutagenesis oligomers) are synthesized with a sequence complementary to stretches of 15 bases on either side of the mutation site as shown in the following diagrams, wherein N indicates complementary bases and M indicates the nucleic acid to be inserted, deleted or substituted. Insertions or deletions ordinarily are made in groups of 3 in order to retain the downstream portion of the gene in phase.

| For deletions: | oligomer DNA<br>vector DNA | $(N)_{15}$ $(N)_{15}$<br>$(N)_{15}$ $(M)$ $(N)_{15}$ |
|---|---|---|
| For substitutions: | oligomer DNA<br>vector DNA | $(N)_{15}$ $(M_1)$ $(N)_{15}$<br>$(N)_{15}$ $(M_2)$ $(N)_{15}$ |
| For insertions: | oligomer DNA<br>vector DNA | $(N)_{15}$ $(M)$ $(N)_{15}$<br>$(N)_{15}$ $(N)_{15}$ |

$M_1$ indicates a base or an oligomer which is not complementary to the base or oligomer $M_2$. Here, $M_1$ is the desired mutant sequence. Ordinarily, oligomers are also prepared that act to make more than one type of mutation at a time.

The antisense oligomer for the Arg-his 32 mutant is p CAG GAG GGC ATT GGC ATG GCG GTT CAG CCA CTG-OH.

The antisense oligomer for the His 73 deletion is p GTG GGT GAG GAG CAC GGT GGA GGG GCA GCC-OH.

The antisense oligomer for the Leu 158 insertion is p TGT TCG TCC TCC TCA AAG CAG GGC AAT GAT CCC-OH.

These primers are synthesized by conventional methods. For use in the mutagenesis procedure, 10 pmoles of the oligomers or lac primer 5'-GTTTTCCCAGTCACGAC-3' are each phosphorylated for 30 min at 37°C in 10 $\mu$l of 50 mM Tris-HCl (pH 7.5), 0.1 mM EDTA, 10 mM MgCl$_2$. 10 mM dithiothreitol, 0.1 mM ATP, containing 2 U of T4 polynucleotide kinase. For use as probes (see infra), 2 pmoles of the synthetic oligonucleotides are phosphorylated as above except that 0.1 mM ATP was replaced with 1 $\mu$M $\gamma$-$^{32}$P ATP (Amersham). Specific activities are routinely higher than 5 X 10$^6$ cpm/pmole of oligonucleotide chain.

Hybridization of each oligomer and the lac primer to the single-stranded DNA from phage M13 mp8/TNF, followed by primer extension, results in the formation of partial heteroduplex DNAs, one strand of which contains the mutant DNA.

For partial heteroduplex formation, single-stranded M13 mp8/TNF DNA (300 ng) is heated to 80°C (2 min), 50°C (5 min), and room temperature (5 min) in 20 $\mu$l 10 mM Tris-HCl (pH 7.5), 0.1 mM EDTA, 50 mM NaCl, containing 1 pmole each of phosphorylated oligomer and primer (added as aliquots from the kinase reaction). Primer extension is started by the addition of 30 $\mu$l 50 mM Tris-HCl (pH 8.0), 0.1 mM EDTA, 12 mM MgCl$_2$, 10 mM dithiothreitol, 0.7 mM ATP, 0.07 mM dATP, 0.2 mM each of dGTP, dTTP, dCTP, and containing 2 U E. coli DNA polymerase I, large fragment and 20 U T4 DNA ligase. After 30 min at room temperature, reaction mixtures are incubated for 4 hr at 37°C followed by overnight incubation at 4°C. Aliquots are phenol-extracted and DNA is precipitated with ethanol and dissolved in 15 $\mu$l of water. DNA in these aliquots is used to transform E. coli JM103.

The lac primer hybridizes to the phage at a location 5' to the oligomer. Primer elongation stabilizes the heteroduplex structure. The oligomer and primer are enzymatically phosphorylated to allow the T4 DNA ligase to join connecting DNA chains.

Phenol-extracted heteroduplex DNA from aliquot C (10 $\mu$l) is added to 10 $\mu$l 0.06 M Na-acetate pH 4.5, 0.6 M NaCl, 0.6 mM ZnCl$_2$, and containing 200 U S$_1$ nuclease. After incubation at 37°C for 5 min, yeast tRNA (5$\mu$g) is added and nucleic acids are recovered by phenol extraction and ethanol precipitation. Using the same S$_1$ conditions, 30 ng of single-stranded M13 mp8 DNA (about 10,000 plaque-forming units) yields less than 100 plaques in a DNA transformation assay, whereas the same amount of RF-DNA retains more than 80 percent of its transforming properties. S$_1$-treated DNA is used to transform E. coli JM103 and the resulting phage analyzed by in situ plaque screening.

Bacterial plates (15-cm diameter) containing several hundred recombinant M13 phage plaques are screened by in situ plaque hybridization (Benton et al., 1977, "Science" 196: 180-182) for both the parental and the mutated genotype using the appropriate labelled oligomers on separate sets of filters (about 10$^6$ cpm per filter). Hybridization is overnight at 50°C, 40 percent formamide, 5 X SSC. Filters are washed at 45°C, 2 X SSC, 0.02 percent sodium dodecyl sulfate, air-dried, and exposed to X-ray film at -70°C using an intensifying screen.

It will be necessary to vary the stringency of the hybridization procedure (by altering the concentration of SSC) in order to resolve the hybridization of oligomer to the mutant DNA strand (a perfect complement) as opposed to hybridization to the starting DNA; each mutant will vary in its ability to hybridize depending upon the nature and number of bases substituted, deleted or inserted. For example, detecting a mutation in a single base will require high stringency conditions to discriminate between mutant and unmutated parental DNA where the mutation is minor, e.g. deletion of a codon or substitution of 1-3 bases, the hybridization probe should be smaller than the mutating oligomer. Typically this will be a probe of about 14 to 20 bases. The task of screening mutant deletions is facilitated by the use of a probe containing or constituting the deleted sequence to assay for loss of the sequence. This probe fails to hybridize to DNA from a selected plaque one can conclude that the desired loss of the target sequence has occurred.

A plaque that hybridizes with the labelled oligomer is picked and inoculated into E. coli JM103. Single-stranded (ss) DNA is prepared from the supernatant and double-stranded (ds) DNA is prepared from the cell pellet. The ssDNA is used as a template for the dideoxy sequencing of the clone using the M13 universal primer or a synthetic oligomer complementary sequences located 3' of the mutated region of the tumor necrosis factor DNA. Dideoxy sequencing confirms that the recovered plaque contains the mutant DNA. Such phage is designated M13 mp8/TNFmtnt.

M13 mp8/TNFmtnt is digested with EcoRI and HindIII and the TNF-encoding fragment recovered. pTrpXAPTNF is digested with EcoRI and HindIII and the vector fragment recovered. The mutant fragment is then ligated to the vector fragment and the ligation mixture used to transform E. coli W3110, NL106, or 294 (ATCC 31446). The mutant TNF is recovered by the method of Examples 18 or 19. Additional information relating to M13 mutagenesis is provided by U.K. patent application 2,130,219A.

The mutants made in accordance with this method are divided into three classes: Substitutions, deletions and insertions, and further subdivided as shown in the following Table. Unless otherwise stated the mutants are of the mature tumor necrosis factor of Fig. 10.

| Mutant Type | | TNF Site | Representative Modification at Indicated Site |
|---|---|---|---|
| A. SUBSTITUTIONS | | | |
| I. | Modifications in the degree or nature of charge | | |
| | 1. | arg 6 | his |
| | 2. | lys 65 | arg |
| | 3. | pro 20 | arg |
| | 4. | asp 10 | his |
| | 5. | glu 53 | thr |
| | 6. | gln 47 | asp |
| | 7. | asp 45 | gln or asn |
| | 8. | asn 39 | asp |
| | 9. | asn 34 | gln |
| | 10. | leu 29 | asp |
| | 11. | tyr 115 | ile |
| | 12. | glu 116 | lys |
| | 13. | pro 117 | thr |
| | 14. | glu 127 | tyr |
| | 15. | lys 128 | his |
| | 16. | ala 134 | tyr |
| | 17. | glu 135 | lys |
| | 18. | tyr 141 | pro |
| | 19. | asp 143 | ser |
| | 20. | ala 145 | thr |
| | 21. | glu 146 | asn |
| | 22. | gln 149 | lys |
| | 23. | leu 120 | lys |
| II. | Modifications in Hydrophilic or Hydrophobic Character | | |
| | 1. | leu 57 | tyr |
| | 2. | ser 52 | leu |
| | 3. | val 41 | tyr |
| | 4. | gly 108 | phe |

29

| Mutant Type | TNF Site | Representative Modification at Indicated Site |
|---|---|---|
| 5. | leu 120 | thr |
| 6. | ser 133 | gly |
| 7. | ala 134 | thr |
| 8. | gly 148 | ser |
| 9. | val 16 | thr |

III. Steric Modification
(changes in side chain bulk)

| | | |
|---|---|---|
| 1. | Leu 63 | phe |
| 2. | Ser 52 | tyr |
| 3. | ile 58 | leu |
| 4. | gly 40 | ile |
| 5. | val 13 | phe |
| 6. | leu 120 | phe |
| 7. | ile 146 | gly |
| 8. | asn 137 | glu |
| 9. | ile 154 | phe |
| 10. | ile 155 | gly |
| 11. | phe 144 | ile |

B. INSERTIONS

| | | |
|---|---|---|
| 1. | after Leu 157 | gly gly-COOH |
| 2. | between Asp 10 and Lys 11 | his |
| 3. | between ile 58 and tyr 59 | leu |
| 4. | between Ser 60 and gln 61 | lys |
| 5. | between arg 31 and arg 32 | ala |
| 6. | between gly 121 and gly 122 | gly |
| 7. | before val 1 | immunogenic polypeptide |
| 8. | after leu 157 | immunogenic polypeptide |
| 9. | between gln 149 and val 150 | gly gly |
| 10. | between ala-1 and val 1 of preTNF | lys arg |

30

| Mutant Type | TNF Site | Representative Modification at Indicated Site |
|---|---|---|

**C. DELETIONS**

| | | |
|---|---|---|
| 1. | Gln 149 | |
| 2. | lys 112 | |
| 3. | val 1 - arg 2 | |
| 4. | val 1 through pro 8 | |
| 5. | ala 22 | |
| 6. | arg 32 | |
| 7. | glu 53 | |
| 8. | ala 111 - lys 112 | |
| 9. | ala 123 | |
| 10. | ile 154 | |
| 11. | glu 127 | |

**D. COMBINATIONS**

| | | |
|---|---|---|
| 1. | ile 58 | leu |
| | leu 57 | phe |
| 2. | gln 149 | delete |
| | tyr 151 | phe |
| 3. | lys 112 | delete |
| | glu 115 | lys |
| 4. | val 1 | thr |
| | ala 22 | lys |
| | gly 24 | asn |
| | ala 33 | asp |
| 5. | tyr 115 | phe |
| | glu 116 | lys |

6. insert gly between gly 121 and gly 122
after leu 157 add gly gly-COOH

7. delete val 1 through gly 66 and substitute
$NH_2$-Leu Ala Ile Ile Gly Phe Tyr Val Gln Gly Ser Glu Ala-
Phe Asp Leu Tyr Asp Pro Arg Asn Ile Glu Ala Ser Leu Arg-
Asp Gly Lys Glu Leu Gln Phe Val Gly Gly Leu Tyr Ile Pro-
Glu Tyr Trp Pro Lys Ala Glu Ala Gly Glu Pro Thr-

| Mutant Type | TNF Site | Representative Modification at Indicated Site |
|---|---|---|
| 8. | delete ala 111 | |
| | ala 109 | gln |
| | leu 120 | his |
| 9. | asn 19 | gln |
| | asn 92 | gln |
| | asn 137 | gln |

Of note are mutations in which trypsin hydrolysis sites at arg 2, arg 6 (see Examples 20 and 21), arg 32 and arg 131 are deleted or modified so as to no longer be susceptible to trypsin. This might extend the half-life of tumor necrosis factor in vivo while reducing the possibility of fermentative clipping. The arg 2 and arg 6 sites are not critical because biological activity is retained even after the regions concerned are removed. However, cleavage at the arg 32 and arg 131 sites leads to activity loses. Accordingly arg 32 and/or arg 131 are desirably substituted with histidinyl or, less preferably, gln. This removes or reduces the enzyme susceptibility of the site but retains the basicity of the side chain. Also, arg 31 is substituted with histidinyl or, less preferably, glutamine for the same reasons. Enzyme hydrolysis sites also are inserted between fusion polypeptides and TNF sequences in order to create sites for predetermined release of mature or mutant TNF, or such sites are substituted for residues within the leader sequence of preTNF.

The substitution of asn at asp 45 and expression in a host cell, (e.g. yeast or mammalian cell), capable of glycosylation is expected to produce a glycosylated tumor necrosis factor.

Example 25

Expression of Tumor Necrosis Factor in Yeast Under the Control of the ADH Promoter

Plasmid TrpXAPTNF is constructed as described in Example 17 except that p20KLT or ptrpETA (or pBR322) is cleaved with EcoRI and HindIII rather than XbaI and HindIII and the synthetic fragment B prepared with an EcoRI cohesive terminus rather than XbaI cohesive terminus. The ligation mixture is then used to transform E. coli ATCC 31446 and a plasmid pTNFRI identified by restriction analysis that contains the tumor necrosis factor encoding DNA bounded by EcoRI sites. Plasmid pTNFRI is isolated, digested with EcoRI and the tumor necrosis factor DNA-containing fragment T-1 recovered.

Plasmid pFRPn (EP 60,057A) is digested with EcoRI, treated with alkaline phosphatase to prevent recircularization, ligated to the T-1 tumor necrosis factor fragment using T4 DNA ligase and the ligation mixture then used to transform E. coli ATCC 31446. Ampicillin resistant colonies yield two series of plasmids having the T-1 insert in opposite orientations as determined by restriction analysis on agarose electrophoresis gels. Plasmids are purified from E. coli transformants and used to transform yeast having the trp1 mutation (for example yeast strain RH218, unrestricted ATCC deposit No. 44076) to the trp+ phenotype. Plasmids oriented such that the start codon of segment T-1 is located adjacent to the alcohol dehydrogenase promoter fragment are found to transform the yeast to tumor necrosis factor expression. Tumor necrosis factor is recovered from extracts of the yeast transformants. The plasmid stability in large scale fermentations is improved by employing an expression plasmid containing the 2 micron origin of replication in place of the pFRPn chromosomal origin of replication (ars 1) and a compatible host strain (J. Beggs, 1978, "Nature" 275: 104-109).

Example 26

Expression of Tumor Necrosis Factor In Mammalian Cells

Plasmid pEHER (EP 117,060A) is digested with EcoRI, treated with calf intestinal alkaline phosphatase, ligated to fragment T-1 from Example 25, and the ligation mixture used to transform E. coli ATCC 31446. Two plasmids are isolated (designated pEHERTNF I and pEHERTNF II) containing the TNF DNA in opposite orientations as determined by restriction analysis on polyacrylamide gels. These plasmids are used to transfect and select CHO DHFR-DUX-B11, CHO 1 and Ltk⁻cells.

Tissue culture cells are transfected by mixing 1 $\mu$g of pEHERTNF I or pEHERTNF II as prepared above with 10 $\mu$g rat carrier DNA in a volume of 250 $\mu$l, 0.25 M $CaCl_2$, followed by dropwise addition of 250 $\mu$l HEPES buffered saline (280 mM MaCl, 1.5 mM $Na_2PO_4$, 50 mM HEPES, pH 7.1). After 30 minutes at room temperature, the solution is added to tissue culture cells growing in 60 mm plastic tissue culture dishes. CHO 1, CHO DHFR-DUX-B11, and Ltk⁻cells are used. The dishes contain 3 ml culture medium appropriate to the host cell.

For CHO 1 and CHO DHFR-DUX-B11 cells, the medium is Ham F-12 media (Gibco) supplemented with 10 percent calf serum, 100 $\mu$/ml penicillin 100 $\mu$g/ml streptomycin, and 2 $\mu$mM L-glutamine. For the Ltk⁻cell line, the medium is Dulbecco modified Eagle's medium (DMEM) supplemented as above.

After 3-16 hours, the medium is removed and the cells are washed with 20 percent glycerol in phosphate buffered saline. Fresh medium is added to each plate and the cells are incubated for 2 more days.

Selection of transfected host cells is carried out by trypsinizing the cells after 2 days growth (which comprises treating the cells with sterile trypsin 0.5 mg/ml containing 0.2 mg/ml EDTA) and adding about $3 \times 10^5$ cells to 10 mm tissue culture plates with selective media. For dhfr⁻ cells the medium is a formulation of (F-12 GIBCO) medium ladking glycine, hypoxanthine, amd thymidine (GHT⁻ medium). For DHFR⁺ host cells, methotrexate (100 - 1000 nM) is added to the normal growth medium. Controls are run using transfection conditions with no plasmid and with plasmid pFD-11 (EP 117,060A) containing normal DHFR. Colonies arising from cells which take up and express the DHFR plasmid are apparent within 1-2 weeks. Transformants are identified that express tumor necrosis factor.

## Claims
**Claims for the following Contracting States : BE, CH, DE, FR, GB, IT, LI, LU, NL, SE**

1. A method for preparing a tumor necrosis factor from an admixture with other proteins, comprising adsorbing the tumor necrosis factor from such admixture onto a substance selected from:
   (i) a silicate,
   (ii) controlled pore glass,
   (iii) alkyl sepharose,
   (iv) particles of an anion exchange resin having substantially uniform particle size,
   and thereafter eluting the tumor necrosis factor.

2. The method of claim 1 wherein the tumor necrosis factor is eluted from controlled pore glass with ethylene glycol.

3. The method of claim 1 wherein a quaternary amino-substituted crosslinked polystyrene anion exchange resin is used.

4. Human tumor necrosis factor which
   a) is unglycosylated;
   b) has a molecular weight of about 17,000 as determined by SDS-PAGE;
   c) is capable of the preferential destruction or growth inhibition of tumor cells when compared to normal cells under the same conditions;
   d) is substantially homogeneous as determined by SDS-PAGE; has a specific activity greater than about 10 million units/mg protein; and is purified to a degree sufficient for sequencing of intact tumor necrosis factor, or a trypsin hydrolysis fragment thereof, by sequential Edman degradation on a spinning cup sequencer equipped with cold traps and using Polybrene® as a carrier in the sequencer cup; and
   e) comprises:

(i) the amino acid sequence 1 to 157 depicted in Fig. 10 hereof; or

(ii) amino acid sequence Glu Thr Pro Glu Gly Ala Glu Ala Lys Pro Trp Tyr Glu Pro; or

(iii) the amino acid sequence of residues 35-66 as depicted in Fig. 10 hereof; or

(iv) the amino acid sequence of residues 110-133 as depicted in Fig. 10 hereof; or

(v) the amino acid sequence of residues 1-6, 7-44, 45-82, 83-90 or 99-157 as depicted in Fig. 10 hereof.

5. The tumor necrosis factor of claim 4 which is free of other cytotoxic proteins.

6. The tumor necrosis factor of claim 5 which is free of a protein selected from lymphotoxin, $\alpha$-globulins, serine proteases and interferons.

7. Tumor necrosis factor which is capable of the preferential destruction or growth inhibition of tumor cells when compared to normal cells under the same conditions, and which consists essentially of the amino acid sequence 1 to 157 depicted in Fig. 10 hereof; optionally with an N-terminal methionyl residue; or a variant (other than rabbit TNF) of the said sequence in which residue $Val^1$ and/or $Arg^2$ thereof is retained.

8. The tumor necrosis factor of claim 7 wherein an amino acid residue has been substituted into, deleted from or inserted into the amino acid sequence 1 to 157 as depicted in Fig. 10 hereof.

9. Tumor necrosis factor which is capable of the preferential destruction or growth inhibition of tumor cells when compared to normal cells under the same conditions, and which comprises a variant of the amino acid sequence 1 to 157 as depicted in Fig. 10 hereof (other than rabbit TNF) in which an amino acid sequence has been substituted, deleted or inserted at a site located within amino acid residues 35 to 66, 110 to 133 or 150 to 157 as depicted in Fig. 10 hereof.

10. Tumor necrosis factor which is capable of the preferential destruction or growth inhibition of tumor cells when compared to normal cells under the same conditions, and which comprises a variant of the amino acid sequence 1 to 157 as depicted in Fig. 10 hereof (other than rabbit TNF) in which an amino acid sequence has been substituted, deleted or inserted at a site located within amino acid residues 67 to 109 as depicted in Fig. 10 hereof.

11. The tumor necrosis factor of any one of claims 8, 9 and 10 wherein the substitution is a substitution of a residue from the classes of neutral, acid, or basic amino acid residues for a residue of the tumor necrosis factor amino acid sequence which is not a member of the class to which the substituted amino acid belongs.

12. The tumor necrosis factor of any one of claims 8, 9 and 10 wherein the substitution is one in which a residue having a sterically bulky side chain replaces a residue not having such a side chain.

13. The tumor necrosis factor of claim 12 wherein the residue not having a sterically bulky side chain is glycine or serine.

14. The tumor necrosis factor of claim 12 or claim 13 wherein the residue having such a side chain is phenylalanine or tryptophan.

15. The tumor necrosis factor of any one of claims 8, 9 and 10 wherein a lysine or arginine residue is deleted or substituted for by an amino acid residue other than lysine and arginine.

16. The tumor necrosis factor of any one of claims 4 to 15 in admixture with a physiologically acceptable buffer, amino acid or nonionic surfactant, or mixtures thereof.

17. The tumor necrosis factor of claim 16 which is lyophilized.

18. DNA encoding tumor necrosis factor of any one of claims 7 to 15.

19. The DNA of claim 18 wherein said DNA is synthetic.

**20.** The DNA of claim 18 or claim 19 present in a replicable vector.

**21.** The DNA of claim 20 wherein the vector is a plasmid or virus.

**22.** A cell transformed with the DNA of claim 20 or claim 21.

**23.** A method comprising culturing a cell transformed with DNA of claim 18 so as to effect expression of that DNA, allowing the tumor necrosis factor to accumulate in the culture and recovering it from the culture.

**24.** A composition comprising tumor necrosis factor of any one of claims 4 to 15 and a component from a non-human cell, which component is physiologically acceptable upon administration to patients.

**25.** The composition of claim 24 wherein said component is a prokaryotic protein.

**26.** A composition comprising cells containing therein heterologous tumor necrosis factor of any one of claims 7 to 15.

**27.** The composition of claim 26 wherein the cells are prokaryotic or lower eukaryotic.

**28.** A composition suitable for the treatment of tumors comprising a tumor necrosis factor according to any of claims 4-17 or as obtainable by the method of any of claims 1-3, and an interferon.

**29.** The composition of claim 28 which is free of other cytotoxic peptides.

**30.** The composition of claim 28 or claim 29 which is free of other plasma proteins.

**31.** A cell free composition suitable for the treatment of tumors comprising a tumor necrosis factor according to any of claims 4-17 or as obtainable by the method of any of claims 1-3, and an interferon, which composition is free of lymphotoxin.

**32.** The composition of any one of claims 28 to 31 wherein the interferon is gamma interferon.

**33.** The composition of any one of claims 28 to 32 which is lyophilised.

**Claims for the following Contracting State : AT**

**1.** A method for preparing a tumor necrosis factor from an admixture with other proteins, comprising adsorbing the tumor necrosis factor from such admixture onto a substance selected from:
(i) a silicate,
(ii) controlled pore glass,
(iii) alkyl sepharose,
(iv) particles of an anion exchange resin having substantially uniform particle size, and
thereafter eluting the tumor necrosis factor.

**2.** The method of claim 1 wherein the tumor necrosis factor is eluted from controlled pore glass with ethylene glycol.

**3.** The method of claim 1 wherein a quaternary amino-substituted crosslinked polystyrene anion exchange resin is used.

**4.** A process which comprises the preparation of human tumor necrosis factor which
a) is unglycosylated;
b) has a molecular weight of about 17,000 as determined by SDS-PAGE:
c) is capable of the preferential destruction or growth inhibition of tumor cells when compared to normal cells under the same conditions;
d) is substantially homogeneous as determined by SDS-PAGE; has a specific activity greater than about 10 million units/mg protein; and is purified to a degree sufficient for sequencing of intact tumor

35

necrosis factor, or a trypsin hydrolysis fragment thereof, by sequential Edman degradation on a spinning cup sequencer equipped with cold traps and using Polybrene® as a carrier in the sequencer cup; and

e) comprises:

(i) the amino acid sequence 1 to 157 depicted in Fig. 10 hereof; or

(ii) amino acid sequence Glu Thr Pro Glu Gly Ala Glu Ala Lys Pro Trp Tyr Glu Pro; or

(iii) the amino acid sequence of residues 35-66 as depicted in Fig. 10 hereof; or

(iv) the amino acid sequence of residues 110-133 as depicted in Fig. 10 hereof; or

(v) the amino acid sequence of residues 1-6, 7-44, 45-82, 83-90 or 99-157 as depicted in Fig. 10 hereof.

5. A process according to claim 4 wherein the tumor necrosis factor is free of other cytotoxic proteins.

6. A process according to claim 5 wherein the tumor necrosis factor is free of a protein selected from lymphotoxin, α-globulins, serine proteases and interferons.

7. A process which comprises the preparation of a tumor necrosis factor which is capable of the preferential destruction or growth inhibition of tumor cells when compared to normal cells under the same conditions, and which consists essentially of the amino acid sequence 1 to 157 depicted in Fig. 10 hereof; optionally with an N-terminal methionyl residue; or a variant (other than rabbit TNF) of the said sequence in which residue $Val^1$ and/or $Arg^2$ thereof is retained.

8. A process according to claim 7 wherein the tumor necrosis factor has an amino acid residue substituted into, deleted from or inserted into the amino acid sequence 1 to 157 as depicted in Fig. 10 hereof.

9. A process which comprises the preparation of a tumor necrosis factor which is capable of the preferential destruction or growth inhibition of tumor cells when compared to normal cells under the same conditions, and which comprises a variant of the amino acid sequence 1 to 157 as depicted in Fig. 10 hereof (other than rabbit TNF) in which an amino acid sequence has been substituted, deleted or inserted at a site located within amino acid residues 35 to 66, 110 to 133 or 150 to 157 as depicted in Fig. 10 hereof.

10. A process which comprises the preparation of a tumor necrosis factor which is capable of the preferential destruction or growth inhibition of tumor cells when compared to normal cells under the same conditions, and which comprises a variant of the amino acid sequence 1 to 157 as depicted in Fig. 10 hereof (other than rabbit TNF) in which an amino acid sequence has been substituted, deleted or inserted at a site located within amino acid residues 67 to 109 as depicted in Fig. 10 hereof.

11. A process according to any one of claims 8, 9 and 10 wherein the tumor necrosis factor has a substitution of a residue from the classes of neutral, acid, or basic amino acid residues for a residue of the tumor necrosis factor amino acid sequence which is not a member of the class to which the substituted amino acid belongs.

12. A process according to any one of claims 8, 9 and 10 wherein the substitution is one in which a residue having a sterically bulky side chain replaces a residue not having such a side chain.

13. A process according to claim 12 wherein the residue not having a sterically bulky side chain is glycine or serine.

14. A process according to claim 12 or claim 13 wherein the residue having such a side chain is phenylalanine or tryptophan.

15. A process according to any one of claims 8, 9 and 10 wherein a lysine or arginine residue is deleted or substituted for by an amino acid residue other than lysine and arginine.

16. A process according to any one of claims 4 to 15 wherein the tumor necrosis factor is in admixture with a physiologically acceptable buffer, amino acid or nonionic surfactant, or mixtures thereof.

EP 0 168 214 B1

**17.** A process according to claim 16 wherein the tumor necrosis factor is lyophilized.

**18.** A process which comprises the preparation of DNA encoding tumor necrosis factor which can be prepared in accordance with any one of claims 7 to 15.

**19.** A process which comprises the preparation of the DNA of claim 18 wherein said DNA is synthetic.

**20.** A process which comprises the preparation of the DNA of claim 18 or claim 19 present in a replicable vector.

**21.** A process which comprises the preparation of the DNA of claim 20 wherein the vector is a plasmid or virus.

**22.** A process which comprises the preparation of a cell transformed with the DNA which can be prepared in accordance with claim 20 or claim 21.

**23.** A method comprising culturing a cell transformed with DNA of claim 18 so as to effect expression of that DNA, allowing the tumor necrosis factor to accumulate in the culture and recovering it from the culture.

**24.** A process which comprises the preparation of a composition having tumor necrosis factor which can be prepared in accordance with any one of claims 4 to 15 and a component from a non-human cell, which component is physiologically acceptable upon administration to patients.

**25.** A process according to claim 24 wherein said component is a prokaryotic protein.

**26.** A process which comprises the preparation of a composition comprising cells containing therein heterologous tumor necrosis factor which can be prepared in accordance with any one of claims 7 to 15.

**27.** A process according to claim 26 wherein the cells are prokaryotic or lower eukaryotic.

**28.** A process which comprises the preparation of a composition suitable for the treatment of tumors which composition has a tumor necrosis factor which can be prepared in accordance with claims 4 to 17 or as obtainable by the method of any of claims 1 to 3, and interferon.

**29.** A process according to claim 28 when the composition is free of other cytotoxic peptides.

**30.** A process according to claim 28 or claim 29 wherein the composition is free of other plasma proteins.

**31.** A process which comprises the preparation of a cell free composition suitable for the treatment of tumors which composition has a tumor necrosis factor which can be prepared in accordance with any of claims 4 to 17 or as obtainable by the method of any of claims 1 to 3, and an interferon, and which is free of lymphotoxin.

**32.** A process according to any one of claims 28 to 31 wherein the interferon is gamma interferon.

**33.** A process according to any one of claims 28 to 32 which is lyophilised.

**Patentansprüche**
**Patentansprüche für folgende Vertragsstaaten : BE, CH, DE, FR, GB, IT, LI, LU, NL, SE**

**1.** Verfahren zur Herstellung eines Tumornekrosefaktors aus einer Mischung davon mit anderen Proteinen, umfassend das Adsorbieren des Tumornekrosefaktors aus einer solchen Mischung an einer Substanz ausgewählt aus:
   (i) einem Silikat,
   (ii) Glas mit abgestimmten Poren,
   (iii) Alkylsepharose,

37

(iv) Teilchen eines Anionenaustauschharzes mit im wesentlichen einheitlicher Teilchengröße, und das anschließende Eluieren des Tumornekrosefaktors.

2. Verfahren nach Anspruch 1, worin der Tumornekrosefaktor mit Äthylenglykol aus dem Glas mit abgestimmten Poren eluiert wird.

3. Verfahren nach Anspruch 1, worin ein quaternäres, aminosubstituiertes, vernetztes Polystyrol-Anionenaustauschharz verwendet wird.

4. Menschlicher Tumornekrosefaktor, der
    a) unglykosyliert ist;
    b) ein durch SDS-PAGE ermitteltes Molekulargewicht von etwa 17.000 aufweist;
    c) im Vergleich zu normalen Zellen unter den gleichen Bedingungen zur präferentiellen Zerstörung oder Wachstumgshemmung von Tumorzellen fähig ist;
    d) im wesentlichen homogen ist, wie durch SDS-PAGE ermittelt, eine spezifische Aktivität von mehr als etwa 10 Millionen Einheiten/mg Protein aufweist und auf einen Grad gereinigt ist, der ausreicht, intakten Tumornekrosefaktor oder ein Trypsinhydrolysefragment davon durch sequentiellen Edman-Abbau auf einem mit einer Kühlfalle ausgestatteten Drehschalen-Sequenzierer und unter Verwendung von Polybrene$^R$ als Träger in der Sequenziererschale zu sequenzieren; und
    e) folgendes umfaßt:
        (i) die in beiliegender Fig.10 dargestellte Aminosäuresequenz 1 bis 157; oder
        (ii) die Aminosäuresequenz Glu Thr Pro Glu Gly Ala Glu Ala Lys Pro Trp Tyr Glu Pro; oder
        (iii) die in beiliegender Fig.10 dargestellte Aminosäuresequenz der Reste 35-66; oder
        (iv) die in beiliegender Fig.10 dargestellte Aminosäuresequenz der Reste 110-133; oder
        (v) die in beiliegender Fig.10 dargestellte Aminosäuresequenz der Reste 1-6, 7-44, 45-82, 83-90, oder 99-157.

5. Tumornekrosefaktor nach Anspruch 4, der frei von anderen cytotoxischen Proteinen ist.

6. Tumornekrosefaktor nach Anspruch 5, der frei von einem aus Lymphotoxin, $\alpha$-Globulinen, Serinproteasen und Interferonen ausgewählten Protein ist.

7. Tumornekrosefaktor, der im Vergleich zu normalen Zellen unter den gleichen Bedingungen zur präferentiellen Zerstörung oder Wachstumshemmung von Tumorzellen fähig ist und der im wesentlichen aus der in beiliegender Fig.10 dargestellten Aminosäuresequenz 1 bis 157 besteht; gegebenenfalls mit einem N-terminalen Methionylrest, oder eine Variante (ausgenommen Hasen-TNF) der genannten Sequenz, in der Rest Val$^1$ und/oder Arg$^2$ davon nach wie vor vorhanden sind.

8. Tumornekrosefaktor nach Anspruch 7, worin ein Aminosäurerest in der in beiliegender Fig.10 dargestellten Aminosäuresequenz 1 bis 157 substituiert, aus ihr gelöscht oder in sie eingesetzt wurde.

9. Tumornekrosefaktor, der im Vergleich zu normalen Zellen unter den gleichen Bedingungen zur präferentiellen Zerstörung oder Wachstumshemmung von Tumorzellen fänig ist und der eine Variante der in beiliegender Fig.10 dargestellten Aminosäuresequenz 1 bis 157 umfaßt (ausgenommen Hasen-TNF), in der eine Aminosäuresequenz an einer Stelle substituiert, gelöscht oder eingesetzt wurde, die sich innerhalb der Aminosäurereste 35 bis 66, 110 bis 133 oder 150 bis 157, wie in beiliegender Fig.10 dargestellt, befindet.

10. Tumornekrosefaktor, der im Vergleich zu normalen Zellen unter den gleichen Bedingungen zu präferentieller Zerstörung oder Wachstumshemmung von Tumorzellen fähig ist und der eine Variante der in beiliegender Fig.10 dargestellten Aminosäuresequenz 1 bis 157 umfaßt (ausgenommen Hasen-TNF), in der eine Aminosäuresequenz an einer Stelle substituiert, gelöscht oder eingesetzt wurde, die sich innerhalb der Aminoreste 67 bis 109, wie in beiliegender Fig.10 dargestellt, befindet.

11. Tumornekrosefaktor nach einem der Ansprüche 8, 9 und 10, worin die Substitution eine Substitution durch einen Rest aus den Klassen neutraler, saurer oder basischer Aminosäuren für einen Rest der Tumornekrosefaktor-Aminosäuresequenz ist, der kein Mitglied der Klasse ist, dem die substituierende Aminosäure angehört.

**12.** Tumornekrosefaktor nach einem der Ansprüche 8, 9 und 10, worin die Substitution eine ist, in der ein Rest mit einer sterisch voluminösen Seitenkette einen Rest ersetzt, der keine solche Seitenkette aufweist.

**13.** Tumornekrosefaktor nach Anspruch 12, worin der Rest, der keine sterisch voluminöse Seitenkette aufweist, Glycin oder Serin ist.

**14.** Tumornekrosefaktor nach Anspruch 12 oder Anspruch 13, worin der Rest mit einer solchen Seitenkette Phenylalanin oder Tryptophan ist.

**15.** Tumornekrosefaktor nach einem der Ansprüche 8, 9 und 10, worin ein Lysin- oder Argininrest gelöscht oder durch einen anderen Aminosäurerest als Lysin oder Arginin substituiert ist.

**16.** Tumornekrosefaktor nach einem der Ansprüche 4 bis 15 in einer Mischung mit einem physiologisch verträglichen Puffer, Aminosäure oder nichtionischem Tensid, oder mit Mischungen davon.

**17.** Tumornekrosefaktor nach Anspruch 16, der lyophilisiert ist.

**18.** DNA, die für Tumornekrosefaktor nach einem der Ansprüche 7 bis 15 kodiert.

**19.** DNA nach Anspruch 18, worin genannte DNA synthetisch ist.

**20.** DNA nach Anspruch 18 oder 19, die in einem replizierbaren Vektor vorhanden ist.

**21.** DNA nach Anspruch 20, worin der Vektor ein Plasmid oder Virus ist.

**22.** Zelle, die mit der DNA nach Anspruch 20 oder Anspruch 21 transformiert ist.

**23.** Verfahren umfassend das Züchten einer mit der DNA nach Anspruch 18 transformierten Zelle, um die Expression dieser DNA zu bewirken, wodurch sich der Tumornekrosefaktor in der Kultur ansammeln kann, und dessen Gewinnen aus dieser Kultur.

**24.** Zusammensetzung umfassend den Tumornekrosefaktor nach einem der Ansprüche 4 bis 15 und eine Komponente einer nichtmenschlichen Zelle, welche Komponente bei Verabreichung an Patienten physiologisch verträglich ist.

**25.** Zusammensetzung nach Anspruch 24, worin genannte Komponente ein prokaryotisches Protein ist.

**26.** Zusammensetzung umfassend Zellen, die heterologen Tumornekrosefaktor nach einem der Ansprüche 7 bis 15 darin enthalten.

**27.** Zusammensetzung nach Anspruch 26, worin die Zellen prokaryotisch oder nieder-eukaryotisch sind.

**28.** Zusammensetzung, die sich zur Behandlung von Tumoren eignet, umfassend einen Tumornekrosefaktor nach einem der Ansprüche 4 bis 17, oder der durch das Verfahren nach einem der Ansprüche 1 bis 3 gewonnen werden kann, sowie ein Interferon.

**29.** Zusammensetzung nach Anspruch 28, die frei von anderen cytotoxischen Peptiden ist.

**30.** Zusammensetzung nach Anspruch 28 oder Anspruch 29, die frei von anderen Plasmaproteinen ist.

**31.** Zellfreie Zusammensetzung, die sich zur Behandlung von Tumoren eignet, umfassend einen Tumornekrosefaktor nach einem der Ansprüche 4 bis 17, oder der durch das Verfahren nach einem der Ansprüche 1 bis 3 gewonnen werden kann, sowie ein Interferon, welche Zusammensetzung frei von Lymphotoxin ist.

**32.** Zusammensetzung nach einem der Ansprüche 28 bis 31, worin das Interferon Gamma-Interferon ist.

**33.** Zusammensetzung nach einem der Ansprüche 28 bis 32, die lyophilisiert ist.

**Patentansprüche für folgenden Vertragsstaat : AT**

**1.** Verfahren zur Herstellung eines Tumornekrosefaktors aus einer Mischung davon mit anderen Proteinen, umfassend das Adsorbieren des Tumornekrosefaktors aus einer solchen Mischung an einer Substanz ausgewählt aus:

(i) einem Silikat,

(ii) Glas mit abgestimmten Poren,

(iii) Alkylsepharose,

(iv) Teilchen eines Anionenaustauschharzes mit einer im wesentlichen einheitlichen Teilchengröße, und das anschließende Eluieren des Tumornekrosefaktors.

**2.** Verfahren nach Anspruch 1, worin der Tumornekrosefaktor mit Äthylenglykol aus dem Glas mit abgestimmten Poren eluiert wird.

**3.** Verfahren nach Anspruch 1, worin ein quaternäres, aminosubstituiertes, vernetztes Polystyrol-Anionenaustauschharz verwendet wird.

**4.** Verfahren, das die Herstellung von menschlichem Tumornekrosefaktor umfaßt, der

a) unglykosyliert ist;

b) ein durch SDS-PAGE ermitteltes Molekulargewicht von etwa 17.000 aufweist;

c) im Vergleich zu normalen Zellen unter den gleichen Bedingungen zur präferentiellen Zerstörung oder Wachstumshemmung von Tumorzellen fähig ist;

d) im wesentlichen homogen ist, wie durch SDS-PAGE ermittelt, eine spezifische Aktivität von mehr als etwa 10 Millionen Einheiten/mg Protein aufweist und auf einen Grad gereinigt ist, der ausreicht,intakten Tumornekrosefaktor oder ein Trypsinhydrolysefragment davon durch sequentiellen Edman-Abbau auf einem mit einer Kühlfalle ausgestatteten Drehschalen-Sequenzierer und unter Verwendung von Polybrene$^R$ als Träger in der Sequenziererschale zu sequenzieren; und

e) folgendes umfaßt:

(i) die in beiliegender Fig.10 dargestellte Aminosäuresequenz 1 bis 157; oder

(ii) die Aminosäuresequenz Glu Thr Pro Glu Gly Ala Glu Ala Lys Pro Trp Tyr Glu Pro; oder

(iii) die in beiliegender Fig.10 dargestellte Aminosäuresequenz der Reste 35-66; oder

(iv) die in beiliegender Fig.10 dargestellte Aminosäuresequenz der Reste 110-133; oder

(v) die in beiliegender Fig.10 dargestellte Aminosäuresequenz der Reste 1-6, 7-44, 45-82, 83-90, oder 99-157.

**5.** Verfahren nach Anspruch 4, worin der Tumornekrosefaktor frei von anderen cytotoxischen Proteinen ist.

**6.** Verfahren nach Anpsruch 5, worin der Tumornekrosefaktor frei von einem aus Lymphotoxin, $\alpha$-Globulinen, Serinproteasen und Interferonen ausgewählten Protein ist.

**7.** Verfahren, das die Herstellung eines Tumornekrosefaktors umfaßt, der im Vergleich zu normalen Zellen unter den gleichen Bedingungen zur präferentiellen Zerstörung oder Wachstumshemmung von Tumorzellen fähig ist und der im wesentlichen aus der in beiliegender Fig.10 dargestellten Aminosäuresequenz 1 bis 157 besteht; gegebenenfalls mit einem N-terminalen Methionylrest; oder eine Variante (ausgenommen Hasen-TNF) der genannten Sequenz, in der Rest Val$^1$ und/oder Arg$^2$ davon nach wie vor vorhanden sind.

**8.** Verfahren nach Anspruch 7, worin der Tumornekrosefaktor einen Aminosäurerest aufweist, der in der in beiliegender Fig.10 dargestellten Aminosäuresequenz 1 bis 157 substituiert, aus ihr gelöscht oder in sie eingesetzt wurde.

**9.** Verfahren, das die Herstellung eines Tumornekrosefaktors umfaßt, der im Vergleich zu normalen Zellen unter den gleichen Bedingungen zur präferentiellen Zerstörung oder Wachstumshemmung von Tumorzellen fänig ist und der eine Variante der in beiliegender Fig.10 dargestellten Aminosäuresequenz 1 bis 157 umfaßt (ausgenommen Hasen-TNF), in der eine Aminosäuresequenz an einer Stelle substituiert, gelöscht oder eingesetzt wurde, die sich innerhalb der Aminosäurereste 35 bis 66, 110 bis 133 oder 150 bis 157, wie in beiliegender Fig.10 dargestellt, befindet.

10. Verfahren, das die Herstellung eines Tumornekrosefaktors umfaßt, der im Vergleich zu normalen Zellen unter den gleichen Bedingungen zu präferentieller Zerstörung oder Wachstumshemmung von Tumorzellen fähig ist und der eine Variante der in beiliegender Fig.10 dargestellten Aminosäuresequenz 1 bis 157 umfaßt (ausgenommen Hasen-TNF), in der eine Aminosäuresequenz an einer Stelle substituiert, gelöscht oder eingesetzt wurde, die sich innerhalb der Aminosäurereste 67 bis 109, wie in beiliegender Fig.10 dargestellt, befindet.

11. Verfahren nach einem der Ansprüche 8, 9 und 10, worin der Tumornekrosefaktor eine Substitution durch einen Rest aus den Klassen neutraler, saurer oder basischer Aminosäuren für einen Rest der Tumornekrosefaktor-Aminosäuresequenz aufweist, der kein Mitglied der Klasse ist, dem die substituierende Aminosäure angehört.

12. Verfahren nach einem der Ansprüche 8, 9 und 10, worin die Substitution eine ist, in der ein Rest mit einer sterisch voluminösen Seitenkette einen Rest ersetzt, der keine solche Seitenkette aufweist.

13. Verfahren nach Anspruch 12, worin der Rest, der keine sterisch voluminöse Seitenkette aufweist, Glycin oder Serin ist.

14. Verfahren nach Anspruch 12 oder Anspruch 13, worin der Rest mit einer solchen Seitenkette Phenylalanin oder Tryptophan ist.

15. Verfahren nach einem der Ansprüche 8, 9 und 10, worin ein Lysin- oder Argininrest gelöscht oder durch einen anderen Aminosäurerest als Lysin oder Arginin substituiert ist.

16. Verfahren nach einem der Ansprüche 4 bis 15, worin sich der Tumornekrosefaktor in einer Mischung davon mit einem physiologisch verträglichen Puffer, Aminosäure oder einem nichtionischen Tensid, oder mit Mischungen davon, befindet.

17. Verfahren nach Anspruch 16, worin der Tumornekrosefaktor lyophilisiert ist.

18. Verfahren, das die Herstellung von DNA umfaßt, die für Tumornekrosefaktor kodiert, der nach einem der Ansprüche 7 bis 15 hergestellt werden kann.

19. Verfahren, das die Herstellung der DNA nach Anspruch 18 umfaßt, worin genannte DNA synthetisch ist.

20. Verfahren, das die Herstellung der DNA nach Anspruch 18 oder 19 umfaßt, die in einem replizierbaren Vektor vorhanden ist.

21. Verfahren, das die Herstellung der DNA nach Anspruch 20 umfaßt, worin der Vektor ein Plasmid oder Virus ist.

22. Verfahren, das die Herstellung einer mit der DNA, die nach Anspruch 20 oder Anspruch 21 hergestellt werden kann, transformierten Zelle umfaßt.

23. Verfahren umfassend das Züchten einer mit der DNA nach Anspruch 18 transformierten Zelle, um die Expression dieser DNA zu bewirken, wodurch sich der Tumornekrosefaktor in der Kultur ansammeln kann, und dessen Gewinnen aus dieser Kultur.

24. Verfahren, das die Herstellung einer Zusammensetzung mit dem Tumornekrosefaktor, der nach einem der Ansprüche 4 bis 15 hergestellt werden kann, und einer Komponente aus einer nichtmenschlichen Zelle umfaßt, welche Komponente bei Verabreichung an Patienten physiologisch verträglich ist.

25. Verfahren nach Anspruch 24, worin die genannte Komponente ein prokaryotisches Protein ist.

26. Verfahren, das die Herstellung einer Zusammensetzung umfaßt, die Zellen umfaßt, die heterologen Tumornekrosefaktor darin enthalten, der nach einem der Ansprüche 7 bis 15 hergestellt werden kann.

**27.** Verfahren nach Anspruch 26, worin die Zellen prokaryotisch oder nieder-eukaryotisch sind.

**28.** Verfahren, das die Herstellung einer Zusammensetzung umfaßt, die sich zur Behandlung von Tumoren eignet, welche Zusammensetzung einen Tumornekrosefaktor, der nach einem der Ansprüche 4 bis 17 hergestellt oder der durch das Verfahren nach einem der Ansprüche 1 bis 3 gewonnen werden kann, sowie ein Interferon aufweist.

**29.** Verfahren nach Anspruch 28, worin die Zusammensetzung frei von anderen cytotoxischen Peptiden ist.

**30.** Verfahren nach Anspruch 28 oder Anspruch 29, worin die Zusammensetzung frei von anderen Plasmaproteinen ist.

**31.** Verfahren, das die Herstellung einer zellfreien Zusammensetzung umfaßt, die sich zur Behandlung von Tumoren eignet, welche Zusammensetzung einen Tumornekrosefaktor, der nach einem der Ansprüche 4 bis 17 hergestellt oder der durch das Verfahren nach einem der Ansprüche 1 bis 3 gewonnen werden kann, sowie ein Interferon aufweist, und frei von Lymphotoxin ist.

**32.** Verfahren nach einem der Ansprüche 28 bis 31, worin das Interferon Gamma-Interferon ist.

**33.** Verfahren nach einem der Ansprüche 28 bis 32, worin die Zusammensetzung lyophilisiert wird.

**Revendications**
**Revendications pour les Etats contractants suivants : BE, CH, DE, FR, GB, IT, LI, LU, NL, SE**

**1.** Procédé de préparation d'un facteur de nécrose tumorale à partir d'un mélange avec d'autres protéines, comprenant l'adsorption du facteur de nécrose tumorale à partir d'un tel mélange sur une substance choisie entre :
   (i) un silicate,
   (ii) un verre à pores ajustés,
   (iii) un Alkyl-Sepharose,
   (iv) des particules d'une résine échangeuse d'anions ayant un diamètre de particules pratiquement uniforme,
puis l'élution du facteur de nécrose tumorale.

**2.** Procédé suivant la revendication 1, dans lequel le facteur de nécrose tumorale est élué du verre à pores ajustés avec de l'éthylène-glycol.

**3.** Procédé suivant la revendication 1, dans lequel est utilisée une résine échangeuse d'anions consistant en un polystyrène réticulé substitué avec un groupe amino quaternaire.

**4.** Facteur de nécrose tumorale humain, qui
   a) est non glycosylé ;
   b) possède un poids moléculaire d'environ 17 000, tel qu'il est déterminé par EGPA-SDS ;
   c) est apte à la destruction ou inhibition de croissance préférentielle de cellules tumorales, comparativement aux cellules normales dans les mêmes conditions ;
   d) est pratiquement homogène, la détermination est effectuée par EGPA-SDS ; possède une activité spécifique supérieure à environ 10 millions d'unités/mg de protéine ; et est purifié à un degré suffisant pour le séquençage du facteur de nécrose tumorale intact, ou d'un de ses fragments d'hydrolyse par la trypsine, par dégradation séquentielle d'Edman sur un séquenceur à coupelle rotative équipé de pièges cryogéniques, avec du Polybrene® comme support dans la coupelle du séquenceur ; et
   e) comprend :
      (i) la séquence d'amino-acides 1 à 157 représentée sur la figure 10 ; ou
      (ii) la séquence d'amino-acides Glu Thr Pro GLu Gly Ala Glu Ala Lys Pro Trp Tyr Glu Pro ; ou
      (iii) la séquence d'amino-acides allant du résidu 35 au résidu 66, représentée sur la figure 10 ; ou
      (iv) la séquence d'amino-acides allant du résidu 110 au résidu 133, représentée sur la figure 10 ; ou

(v) la séquence d'amino-acides correspondant aux résidus 1-6, 7-44, 45-82, 83-90 ou 99-157 représentée sur la figure 10.

**5.** Facteur de nécrose tumorale suivant la revendication 4, qui est dépourvu d'autres protéines cytotoxiques.

**6.** Facteur de nécrose tumorale suivant la revendication 5, qui est dépourvu d'une protéine choisie entre une lymphotoxine, des $\alpha$-globulines, des sérine-protéases et des interférons.

**7.** Facteur de nécrose tumorale qui est apte à la destruction ou l'inhibition de croissance préférentielle de cellules tumorales, comparativement à des cellules normales dans les mêmes conditions, et qui consiste essentiellement en la séquence d'amino-acides 1 à 157 représentée sur la figure 10 ; facultativement avec un résidu méthionyle N-terminal ; ou un variant (autre que le TNF de lapin) de ladite séquence dans lequel son résidu Val$^1$ et/ou Arg$^2$ est retenu.

**8.** Facteur de nécrose tumorale suivant la revendication 7, dans lequel un résidu d'amino-acide a été introduit par substitution dans, éliminé par délétion de, ou inséré dans, la séquence d'amino-acides 1 à 157 représentée sur la figure 10.

**9.** Facteur de nécrose tumorale qui est apte à la destruction ou l'inhibition de croissance préférentielle de cellules tumorales, comparativement à des cellules normales dans les mêmes conditions, et qui comprend un variant de la séquence d'amino-acides 1 à 157 représentée sur la figure 10 (autre que le TNF de lapin) dans lequel une séquence d'amino-acides a été introduite par substitution, éliminée par délétion ou insérée à un site situé à l'intérieur de la séquence de résidus d'amino-acides 35 à 66, 110 à 133 ou 150 à 157 représentée sur la figure 10.

**10.** Facteur de nécrose tumorale qui est apte à la destruction ou à l'inhibition de croissance préférentielle de cellules tumorales, comparativement à des cellules normales dans les mêmes conditions, et qui comprend un variant de la séquence d'amino-acides 1 à 157 représentée sur la figure 10 (autre que le TNF de lapin) dans lequel une séquence d'amino-acides a été introduite par substitution, éliminée par délétion ou insérée à un site situé à l'intérieur de la séquence de résidus d'amino-acides 67 à 109 représentée sur la figure 10.

**11.** Facteur de nécrose tumorale suivant l'une quelconque des revendications 8, 9 et 10, dans lequel la substitution est une substitution d'un résidu de la catégorie des résidus d'amino-acides neutres, acides ou basiques, en remplacement d'un résidu de la séquence d'amino-acides du facteur de nécrose tumorale qui n'est pas un membre de la catégorie à laquelle appartient l'amino-acide de substitution.

**12.** Facteur de nécrose tumorale suivant l'une quelconque des revendications 8, 9 et 10, dans lequel la substitution est une substitution dans laquelle un résidu possédant une chaîne latérale stériquement volumineuse remplace un résidu ne possédant pas une telle chaîne latérale.

**13.** Facteur de nécrose tumorale suivant la revendication 12, dans lequel le résidu ne possédant pas une chaîne latérale stériquement volumineuse est un résidu glycine ou sérine.

**14.** Facteur de nécrose tumorale suivant la revendication 12 ou la revendication 13, dans lequel le résidu possédant une telle chaîne latérale est un résidu phénylalanine ou tryptophane.

**15.** Facteur de nécrose tumorale suivant l'une quelconque des revendications 8, 9 et 10, dans lequel un résidu lysine ou arginine est éliminé par délétion ou introduit en remplacement d'un résidu d'amino-acide autre qu'un résidu lysine ou arginine.

**16.** Facteur de nécrose tumorale suivant l'une quelconque des revendications 4 à 15, en mélange avec un tampon, un amino-acide ou un surfactant non ionique physiologiquement acceptable, ou un de leurs mélanges.

**17.** Facteur de nécrose tumorale suivant la revendication 16, qui est lyophilisé.

**18.** ADN codant pour le facteur de nécrose tumorale suivant l'une quelconque des revendications 7 à 15.

**19.** ADN suivant la revendication 18, qui est un ADN synthétique.

**20.** ADN suivant la revendication 18 ou la revendication 19, présent dans un vecteur réplicable.

**21.** ADN suivant la revendication 20, dans lequel le vecteur est un plasmide ou un virus.

**22.** Cellule formée avec l'ADN suivant la revendication 20 ou la revendication 21.

**23.** Procédé comprenant la culture d'une cellule transformée avec l'ADN suivant la revendication 18 de manière à provoquer l'expression de cet ADN, l'étape consistant à laisser le facteur de nécrose tumorale s'accumuler dans la culture, et la séparation de ce facteur de la culture.

**24.** Composition comprenant le facteur de nécrose tumorale suivant l'une quelconque des revendications 4 à 15 et un constituant provenant d'une cellule non humaine, constituant qui est physiologiquement acceptable par administration à des patients.

**25.** Composition suivant la revendication 24, dans laquelle le constituant est une protéine procaryotique.

**26.** Composition comprenant des cellules renfermant le facteur de nécrose tumorale hétérologue suivant l'une quelconque des revendications 7 à 15.

**27.** Composition suivant la revendication 26, dans laquelle les cellules sont des cellules de procaryotes ou d'eucaryotes inférieurs.

**28.** Composition apte au traitement de tumeurs, comprenant un facteur de nécrose tumorale suivant l'une quelconque des revendications 4 à 17 ou pouvant être obtenu par le procédé suivant l'une quelconque des revendications 1 à 3, et un interféron.

**29.** Composition suivant la revendication 28, qui est dépourvue d'autres peptides cytotoxiques.

**30.** Composition suivant la revendication 28 ou la revendication 29, qui est dépourvue d'autres protéines plasmatiques.

**31.** Composition acellulaire apte au traitement de tumeurs, comprenant un facteur de nécrose tumorale suivant l'une quelconque des revendications 4 à 17 ou pouvant être obtenu par le procédé suivant l'une quelconque des revendications 1 à 3, et un interféron, composition qui est dépourvue de lymphotoxine.

**32.** Composition suivant l'une quelconque des revendications 28 à 31, dans laquelle l'interféron est le gamma-interféron.

**33.** Composition suivant l'une quelconque des revendications 28 à 32, qui est lyophilisée.

**Revendications pour l'Etat contractant suivant : AT**

**1.** Procédé de préparation d'un facteur de nécrose tumorale à partir d'un mélange avec d'autres protéines, comprenant l'adsorption du facteur de nécrose tumorale à partir d'un tel mélange sur une substance choisie entre :
    (i) un silicate,
    (ii) un verre à pores ajustés,
    (iii) un Alkyl-Sepharose,
    (iv) des particules d'une résine échangeuse d'anions possédant un diamètre de particules pratiquement uniforme,
puis l'élution du facteur de nécrose tumorale.

**2.** Procédé suivant la revendication 1, dans lequel le facteur de nécrose tumorale est élué du verre à pores ajustés avec de l'éthylène-glycol.

3. Procédé suivant la revendication 1, dans lequel est utilisée une résine échangeuse d'anions consistant en un polystyrène réticulé substitué avec un groupe amino quaternaire.

4. Procédé qui comprend la préparation d'un facteur de nécrose tumorale humain, qui
   a) est non glycosylé ;
   b) possède un poids moléculaire d'environ 17 000, tel qu'il est déterminé par EGPA-SDS ;
   c) est apte à la destruction ou inhibition de croissance préférentielle de cellules tumorales, comparativement à des cellules normales dans les mêmes conditions ;
   d) est pratiquement homogène, la détermination étant effectuée par EGPA-SDS ; possède une activité spécifique supérieure à environ 10 millions d'unités/mg de protéine ; et est purifié à un degré suffisant pour le séquençage du facteur de nécrose tumorale intacte, ou d'un de ses fragments d'hydrolyse par la trypsine, par dégradation séquentielle d'Edman sur un séquenceur à coupelle rotative équipé de pièges cryogéniques, au moyen de Polybrene® comme support dans la coupelle du séquenceur ; et
   e) comprend :
      (i) la séquence d'amino-acides 1 à 157 représentée sur la figure 10 ; ou
      (ii) la séquence d'amino-acides Glu Thr Pro GLu Gly Ala Glu Ala Lys Pro Trp Tyr Glu Pro ; ou
      (iii) la séquence d'amino-acides allant du résidu 35 au résidu 66, représentée sur la figure 10 ; ou
      (iv) la séquence d'amino-acides allant du résidu 110 au résidu 133, représentée sur la figure 10 ; ou bien
      (v) la séquence d'amino-acides correspondant aux résidus 1-6, 7-44, 45-82, 83-90 ou 99-157 représentée sur la figure 10.

5. Procédé suivant la revendication 4, dans lequel le facteur de nécrose tumorale est dépourvu d'autres protéines cytotoxiques.

6. Procédé suivant la revendication 5, dans lequel le facteur de nécrose tumorale est dépourvu d'une protéine choisie entre une lymphotoxine, des $\alpha$-globulines, des sérine-protéases et des interférons.

7. Procédé qui comprend la préparation d'un facteur de nécrose tumorale qui est apte à la destruction ou l'inhibition de croissance préférentielle de cellules tumorales, comparativement à des cellules normales dans les mêmes conditions, et qui consiste essentiellement en la séquence d'amino-acides 1 à 157 représentée sur la figure 10 ; facultativement avec un résidu méthionyle N-terminal ; ou un variant (autre que le TNF de lapin) de ladite séquence dans lequel son résidu Val$^1$ et/ou Arg$^2$ est retenu.

8. Procédé suivant la revendication 7, dans lequel le facteur de nécrose tumorale possède un résidu d'amino-acide introduit par substitution dans, éliminé par délétion de ou inséré dans, la séquence d'amino-acides 1 à 157 représentée sur la figure 10.

9. Procédé qui comprend la préparation d'un facteur de nécrose tumorale qui est apte à la destruction ou l'inhibition de croissance préférentielle de cellules tumorales, comparativement à des cellules normales dans les mêmes conditions, et qui comprend un variant de la séquence d'amino-acides 1 à 157 représentée sur la figure 10 (autre que le TNF de lapin) dans lequel une séquence d'amino-acides a été introduite par substitution, éliminée par délétion ou insérée à un site situé à l'intérieur de la séquence des résidus d'amino-acides 35 à 66, 110 à 133 ou 150 à 157 représentée sur la figure 10.

10. Procédé qui comprend la préparation d'un facteur de nécrose tumorale qui est apte à la destruction ou l'inhibition de croissance préférentielle de cellules tumorales, comparativement à des cellules normales dans les mêmes conditions, et qui comprend un variant de la séquence d'amino-acides 1 à 157 représentée sur la figure 10 (autre que le TNF de lapin) dans lequel une séquence d'amino-acides a été introduite par substitution, éliminée par délétion ou insérée à un site situé à l'intérieur de la séquence des résidus d'amino-acides 67 à 109 représentée sur la figure 10.

11. Procédé suivant l'une quelconque des revendications 8, 9 et 10, dans lequel le facteur de nécrose tumorale possède une substitution d'un résidu de la catégorie des résidus d'amino-acides neutres, acides ou basiques, en remplacement d'un résidu de la séquence d'amino-acides de facteur de nécrose tumorale qui n'est pas un membre de la catégorie à laquelle appartient l'amino-acide de substitution.

**12.** Procédé suivant l'une quelconque des revendications 8, 9 et 10, dans lequel la substitution est une substitution dans laquelle un résidu possédant une chaîne latérale stériquement volumineuse remplace un résidu ne possédant pas une telle chaîne latérale.

**13.** Procédé suivant la revendication 12, dans lequel le résidu ne possédant pas une chaîne latérale stériquement volumineuse est un résidu glycine ou sérine.

**14.** Procédé suivant la revendication 12 ou la revendication 13, dans lequel le résidu possédant une telle chaîne latérale est un résidu phénylalanine ou tryptophane.

**15.** Procédé suivant l'une quelconque des revendications 8, 9 et 10, dans lequel un résidu lysine ou arginine est éliminé par délétion ou remplacé par un résidu d'amino-acide autre qu'un résidu lysine ou arginine.

**16.** Procédé suivant l'une quelconque des revendications 4 à 15, dans lequel le facteur de nécrose tumorale est présent en mélange avec un tampon, amino-acide ou surfactant non ionique physiologiquement acceptable, ou un de leurs mélanges.

**17.** Procédé suivant la revendication 16, dans lequel le facteur de nécrose tumorale est lyophilisé.

**18.** Procédé qui comprend la préparation d'ADN codant pour un facteur de nécrose tumorale qui peut être préparé suivant l'une quelconque des revendications 7 à 15.

**19.** Procédé qui comprend la préparation de l'ADN suivant la revendication 18, dans lequel ledit ADN est synthétique.

**20.** Procédé qui comprend la préparation de l'ADN suivant la revendication 18 ou la revendication 19, présent dans un vecteur réplicable.

**21.** Procédé qui comprend la préparation de l'ADN suivant la revendication 20, dans lequel le vecteur est un plasmide ou un virus.

**22.** Procédé qui comprend la préparation d'une cellule transformée avec l'ADN qui peut être préparé suivant la revendication 20 ou la revendication 21.

**23.** Procédé comprenant la culture d'une cellule transformée avec l'ADN suivant la revendication 18 de manière à effectuer l'expression de cet ADN, l'étape consistant à laisser le facteur de nécrose tumorale s'accumuler dans la culture, et à séparer ce facteur de la culture.

**24.** Procédé qui comprend la préparation d'une composition comprenant un facteur de nécrose tumorale qui peut être préparé suivant l'une quelconque des revendications 4 à 15 et un constituant provenant d'une cellule non humaine, constituant qui est physiologiquement acceptable par administration à des patients.

**25.** Procédé suivant la revendication 24, dans lequel le constituant est une protéine procaryotique.

**26.** Procédé qui comprend la préparation d'une composition comprenant des cellules renfermant un facteur de nécrose tumorale hétérologue qui peut être préparé suivant l'une quelconque des revendications 7 à 15.

**27.** Procédé suivant la revendication 26, dans lequel les cellules sont des cellules de procaryotes ou d'eucaryotes inférieurs.

**28.** Procédé qui comprend la préparation d'une composition apte au traitement de tumeurs, composition qui comprend un facteur de nécrose tumorale qui peut être préparé suivant l'une quelconque des revendications 4 à 17 ou qui peut être obtenu par le procédé suivant l'une quelconque des revendications 1 à 3, et un interféron.

**29.** Procédé suivant la revendication 28, dans lequel la composition est dépourvue d'autres peptides cytotoxiques.

**30.** Procédé suivant la revendication 28 ou la revendication 29, dans lequel la composition est dépourvue d'autres protéines plasmatiques.

**31.** Procédé qui comprend la préparation d'une composition acellulaire apte au traitement de tumeurs, composition qui comprend un facteur de nécrose tumorale qui peut être préparé suivant l'une quelconque des revendications 4 à 17 ou qui peut être obtenu par le procédé suivant l'une quelconque des revendications 1 à 3, et un interféron, et qui est dépourvue de lymphotoxine.

**32.** Procédé suivant l'une quelconque des revendications 28 à 31, dans lequel l'interféron est le gamma-interféron.

**33.** Procédé suivant l'une quelconque des revendications 28 à 32, qui est lyophilisé.

*Fig.1.*

Elution Profile – Controlled Pore Glass

*Fig. 2.*

Elution Profile-DEAE

## Fig.3.

### Elution Profile-fast Protein Liquid Chromatography

EP 0 168 214 B1

Fig.4.

Elution Profile – Chromatofocusing

# Fig. 5.

## *Fig.6.*

### Elution Profile-HPLC

Fig.7.

EP 0 168 214 B1

## *Fig.8.*

EP 0 168 214 B1

# Fig.9.

### Combined Activity of Tumor Necrosis Factor and Gamma Interferon

MUIFN-γ (Units/ml)

TNF (Units/ml)

BI6 (5000 Cells/well) 72 Hr Incubation

EP 0 168 214 B1

## Fig. 10.

### Amino Acid and Nucleotide Sequence for Human Tumor Necrosis Factor

```
  -20                                              -10                                                1
    ser pro ser pro gln gln phe pro arg asp leu ser leu ile ser pro leu ala gln ala Val Arg Ser Ser Ser Arg Thr Pro Ser
T   TCT CCT TCT CCC CAA CAG TTC CCC AGG GAC CTC TCT CTA ATC AGC CCT CTG GCC CAG GCA GTC AGA TCA TCT TCT CGA ACC CCG AGT

  10                                              20                                                30
Asp Lys Pro Val Ala His Val Val Ala Asn Pro Gln Ala Glu Gly Gln Leu Gln Trp Leu Asn Arg Arg Ala Asn Ala Leu Leu Ala Asn
GAC AAG CCT GTA GCC CAT GTT GTA GCA AAC CCT CAA GCT GAG GGG CAG CTC CAG TGG CTG AAC CGC CGG GCC AAT GCC CTC CTG GCC AAT

  40                                              50                                                60
Gly Val Glu Leu Arg Asp Asn Gln Leu Val Val Pro Ser Glu Gly Leu Tyr Leu Ile Tyr Ser Gln Val Leu Phe Lys Gly Gln Gly Cys
GGC GTG GAG CTG AGA GAT AAC CAG CTG GTG GTG CCA TCA GAG GGC CTG TAC CTC ATC TAC TCC CAG GTC CTC TTC AAG GGC CAA GGC TGC

  70                                              80                                                90|
Pro Ser Thr His Val Leu Leu Thr His Thr Ile Ser Arg Ile Ala Val Ser Tyr Gln Thr Lys Val Asn Leu Leu Ser Ala Ile Lys Ser
CCC TCC ACC CAT GTG CTC CTC ACC CAC ACC ATC AGC CGC ATC GCC GTC TCC TAC CAG ACC AAG GTC AAC CTC CTC TCT GCC ATC AAG AGC

  100                                             110                                               120
Pro Cys Gln Arg Glu Thr Pro Glu Gly Ala Glu Ala Lys Pro Trp Tyr Glu Pro Ile Tyr Leu Gly Gly Val Phe Gln Leu Glu Lys Gly
CCC TGC CAG AGG GAG ACC CCA GAG GGG GCT GAG GCC AAG CCC TGG TAT GAG CCC ATC TAT CTG GGA GGG GTC TTC CAG CTG GAG AAG GGT

  130                                             140                                   150                         157
Asp Arg Leu Ser Ala Glu Ile Asn Arg Pro Asp Tyr Leu Asp Phe Ala Glu Ser Gly Gln Val Tyr Phe Gly Ile Ile Ala Leu OP
GAC CGA CTC AGC GCT GAG ATC AAT CGG CCC GAC TAT CTC GAC TTT GCC GAG TCT GGG CAG GTC TAC TTT GGG ATC ATT GCC CTG TGA GGA

GGACGAACATCCAACCTTCCCAAACGCCTCCCCTGCCCCAATCCCTTTATTACCCCCTCCTTCAGACACCCTCAACCTCTTCTGGCTCAAAAAGAGAATTGGGGGCTTAGGGTCGGAACC

CAAGCTTAGAACTTTAAGCAACAAGACCACCACTTCGAAACCTGGGATTCAGGAATGTGTGGCCTGCACAGTGAATTGCTGGCAACCACTAAGAATTCAAACTGGGGCCTCCAGAACTCA

CTGGGGCCTACAGCTTTGATCCCTGACATCTGGAATCTGGAGACCAGGGAGCCTTTGGTTCTGGCCAGAATGCTGCAGGACTTGAGAAGACCTCACCTAGAAATTGACACAAGTGGACCT

TAGGCCTTCCTCTCTCCAGATGTTTCCAGACTTCCTTGAGACACGGAGCCCAGCCCTCCCCATGGAGCCAGCTCCCTCTATTTATGTTTGCACTTGTGATTATTTATTATTTATTTATTA

TTTATTTATTTACAGATGAATGTATTTATTTGGGAGACCGGGGTATCCTGGGGGACCCAATGTAGGAGCTGCCTTGGCTCAGACATGTTTTCCGTGAAAACGGAGCTGAACAATAGGCTG

TTCCCATGTAGCCCCCTGGCCTCTGTGCCTTCTTTTGATTATGTTTTTTAAAATATTTATCTGATTAAGTTGTCTAAACAATGCTGATTTGGTGACCAACTGTCACTCATTGCTGAGCCT

CTGCTCCCCAGGGGAGTTGTGTCTGTAATCGCCCTACTATTCAGTGGCGAGAAATAAAGTTTGCTT
```

Synthetic Fragment B

met val arg ser ser ser arg thr
5' CTAG AATT ATG GTA CGT TCT TCT TCT CGT ACT
TTAA TAC CAT GCA AGA AGA AGA GCA TGA GGCT 3'

Fig.11.